# EUROPEAN PATENT APPLICATION

(11) **EP 3 214 177 A2**
(43) Date of publication of application: **06.09.2017**
(21) Application number: 17162147.7
(22) Date of filing: 07.04.1999
(51) Int. Cl.: C12N 15/82, C12N 15/11

(54) **METHODS AND MEANS FOR OBTAINING MODIFIED PHENOTYPES**

(30) Priority: 08.04.1998 US 56767; 03.08.1998 US 127735
(62) Divisional of application: 10183703.7
(71) Applicant: Commonwealth Scientific and Industrial Research Organisation, Acton ACT 2601 (AU)
(72) Inventor: WATERHOUSE, Peter, Michael, Paddington, Queensland 4064 (AU); WANG, Ming-Bo, Kaleen, Canberra, Australian Capital Territory 2617 (AU); GRAHAM, Michael, Wayne, San Mateo, CA California 94403 (US)
(74) Representative: Almond-Martin, Carol

(57) **Abstract**

Methods and means are provided for reducing the phenotypic expression of a nucleic acid of interest in eukaryotic cells, particularly plant cells, by introducing a chimeric RNA molecule comprising at least one RNA region with a nucleotide sequence comprising
i. a sense nucleotide sequence of at least 15 consecutive nucleotides having 100% sequence identity with at least part of the nucleotide sequence of the nucleic acid of interest; and
ii. an antisense nucleotide sequence including at least 15 consecutive nucleotides, having about 100% sequence identity with the complement of said at least 15 consecutive nucleotides of said sense nucleotide sequence;
wherein said RNA region is capable of forming an artificial hairpin RNA structure with a double stranded RNA stem by base-pairing between the regions with sense and antisense nucleotide sequence such that said at least 15 consecutive nucleotides of the sense sequence basepair with said at least 15 consecutive nucleotides of the antisense sequence, wherein said nucleic acid of interest is a gene integrated into the genome of said eukaryotic cell, wherein said RNA molecule is produced by transcription of a chimeric gene.

## Description

### FIELD OF THE INVENTION

The invention relates to methods for reducing the phenotypic expression of a nucleic acid sequence of interest in eucaryotic cells, particularly plant cells, by simultaneously providing the cells with chimeric genes encoding sense and anti sense RNA molecules comprising nucleotide sequences respectively homologous and complementary to at least part of the nucleotide sequence of the nucleic acid of interest. The sense and antisense RNA molecules may be provided as one RNA molecule, wherein the sense and antisense RNA may be linked by a spacer nucleotide sequence and are capable of forming a double stranded RNA molecule. In one aspect of the invention, the methods are directed towards reducing viral infection, resulting in extreme virus resistance. In another embodiment the methods are directed towards reducing the phenotypic expression of an endogenous gene in a plant cell. The invention further relates to high throughput screening methods for identifying the phenotype endowed by the nucleic acid of interest in plant cells. Also provided are plant cells comprising such RNA molecules, as well as plants consisting essentially of such plant cells.

### BACKGROUND OF THE INVENTION

In 1985, Sanford and Johnston proposed the concept of parasite-derived resistance. They postulated that key gene products from a parasite expressed in the host in a dysfunctional form, in excess or at a wrong developmental stage, should disrupt the function of the parasite with minimal effect on the host (Sanford & Johnston, 1985). Using the QB bacteriophage as a model, they proposed that expression, in bacteria, of the bacteriophage coat protein or modified replicase or an antisense RNA complementary to the QB genome could all give resistance. They also proposed that such approaches would be applicable, in plants, to plant viruses and particularly the use of a modified plant virus replicase. The expression of the coat protein of the plant virus, tobacco mosaic virus (TMV), in tobacco was the first practical validation of this concept for plant virus resistance. This work (Powell-Abel *et al.,* 1986) showed that the expression of the TMV coat protein, from a transgene under the control of the cauliflower mosaic virus 35S promoter, conferred on the plants resistance to TMV. The same group (Powell *et al.,* 1990) showed that, generally, plants expressing higher levels of coat protein were more resistant to TMV than plants expressing low levels. Since this demonstration there have been very many examples of plants transformed with virus coat protein genes showing resistance (Table 1). There have also been a number of reports of plant virus resistance in plants expressing wild-type replicase (Braun and Hemenway, 1992, Brederode *et al.,* 1995), truncated replicase (Carr *et al.* 1992), modified replicase (Longstaff *et al.* 1993), or antisense viral RNA (Kawchuck *et al.* 1991).

In 1992, Dougherty and colleagues were using different forms of the coat protein gene of tobacco etch virus (TEV) and discovered that some plants containing untranslatable "sense" coat protein genes and antisense coat protein genes showed extreme resistance (ER) to the virus (Lindbo & Dougherty, 1992 a,b). This resistance was functional at the whole plant level and at the single cell level. TEV was unable to replicate in protoplasts derived from ER plants but replicated to high levels in protoplasts from non-transgenic tobacco. Dougherty *et al.* concluded that the mechanism creating the extreme resistance for the untranslatable sense construct was not the same as the often reported coat protein-mediated strategy. They proposed that the mRNA of the untranslatable sense construct was hybridising with the minus sense genome of the virus and interfering with the procession of replication complexes on the minus strand. They suggested that the use of viral sequence that could form intramolecular pairing should be avoided as this would interfere with their ability to hybridise to the target viral RNA.

**Table 1: Plant species that have been genetically engineered for virus resistance (from Rebecca Grumet, Hort Science 30[3] 1995)**

| **Species** | **Viruses** |
|---|---|
| **Tobacco** {tc **"Tobacco** " \1 3} (Nicotiana tabacum L.) | AIMV, ArMV, CMV, PVX, PVY, TEV, TGMV, TMV, TRV, TSV, TSWV |
| **Other Nicotiana spp.** (N. debneyii, N. benthamiana, N. clevelandii) | ACMV, BYMV, CyMV, CyRSV, BCTV, PEBV, PPV, PVS, WMV |
| **Potato** {tc **"Potato** " \1 3} (Solanum tuberusom L.) | PI, RV, PVY |
| **Tomato** {tc **"Tomato** " \1 3} (Lycopersicon esculentum L.) | AIMV, CMV, TMV, TYLCV |
| **Cucumber** {tc **"Cucumber"** \1 3} (Cucumis sativus L.) | CMV |
| **Melon** {tc **"Melon** " \1 3} (Cucumis melo L.) | CMV, ZYMV |
| **Alfalfa** {tc **"Alfalfa** " \1 3} (Medicago sativa L.) | AIMV |
| **Papaya** {tc **"Papaya** " \1 3} (Carica papaya L.) | PRSV |
| **Corn** {tc **"Corn** " \1 3} (Zea mays L.) | MDMV |
| **Rice** {tc **"Rice** " \1 3} (Oryza sativa L.) | RSV |
| **Rapeseed** {tc **"Rapeseed** " \1 3} (Brassica napus L.) | TYMV |

The Dougherty group expanded their investigations to plants containing untranslatable sense potato virus Y (PVY) coat protein genes. They obtained results similar to those with TEV and showed that the plants with ER had high transgene copy number, highly active transcription of the transgenes, and low levels of steady state mRNA from the PVY transgene (Lindbo *et al.* 1993, Smith *et al.* 1994). The following model for this mechanism of the resistance was proposed: the high level of transcription of the viral transgene triggers a cytoplasmic based, post transcriptional cellular surveillance system that targets specific RNAs for elimination. As the transgene encodes a transcript comprising viral sequences the mechanism not only degrades the transgene mRNA but also the same sequences in the viral genomic RNA. A key point in this model is the need for a high level of transcription of the transgene provided by high copy number (3-8; Goodwin *et al.* 1996). Alternatively, the RNA threshold required to trigger the mechanism can be reached by a more modest transcription level aided by the viral RNA from replication in early infection. This gives rise to a "recovery phenotype" where the plant is initially infected and shows symptoms but then produces new growth without virus symptoms and which are extremely resistant to infection.

This proposal was substantiated by the findings that gene silencing of non-viral transgenes could also be due to a post transcriptional mechanism (Ingelbrecht *et al.* 1994; de Carvalho Niebel *et al.* 1995) operating at an RNA level.

A link between non-viral gene silencing and this pathogen derived resistance was provided by inoculating transgenic plants, in which a GUS transgene was known to be silenced by a post transcriptional mechanism, with a virus containing GUS sequences (English *et al.* 1996). In this situation the plants were extremely resistant to the viral infection. However, the same plants were susceptible to the virus if they contained no GUS sequences.

The degree of viral resistance is not always directly related to the level of viral transgene transcription (Mueller *et al.* 1995; English *et al.* 1996) suggesting that there may be an alternative mechanism of inducing the resistance. To accommodate these discrepancies, an alternative model has been proposed in which the crucial factor affecting the resistance is not the level but the quality of the transgene mRNA (English *et al.* 1996). According to this model, the transgene can only induce resistance (or gene silencing) if it is transcribed to produce "aberrant" RNA. There have been many examples of post-transcriptional gene silencing and methylation of the transgene (Hobbs *et al.* 1990; Ingelbrecht *et al.* 1994) and methylation of the transgene has also been found to be associated in some cases of extreme viral resistance (Smith *et al.* 1994, English 1996). In the proposed model, methylation of the transgene leads to the production of aberrant RNAs which induce the cytoplasmic RNA surveillance system. Baulcombe and English have suggested that this method of induction may be the same as that found for the silencing of *met2* in *A.immersus.* In this system transcription of the *met2* RNA was terminated in the methylated regions of the endogenous gene thus producing aberrant truncated RNAs. It was suggested that the methylation was a consequence of ectopic interaction between the transgene and a homologous region of a corresponding region of the endogenous gene (Barry *et al.* 1993). The presence of multiple transgenes would create an increased likelihood of ectopic pairing and is therefore consistent with the correlation between high copy number and extreme viral resistance (Mueller *et al.,* 1995; Goodwin *et al.* 1996; Pang *et al.,* 1996).

This whole area has been reviewed recently (e.g. Baulcombe (1996) and Stam *et al.* (1997)) and several models were presented. All models call for a high degree of sequence specificity because the resistance is very (strain) specific and therefore invoke base pairing interaction with an RNA produced from the transgene. One explanation for the induction of the virus resistance or gene silencing with sense transgenes is that the plant's RNA dependent RNA polymerase generates complementary RNAs using the transgene mRNA as a template (Schiebel *et al.* 1993a,b). This hypothetical complementary RNA (cRNA) has not been detected (Baulcombe 1996) but it is expected that the cRNAs will be small and heterodisperse RNAs rather than full complements (Schiebel 1993ab, Baulcombe1996) and therefore difficult to detect.

The possible methods of action of the cRNA in mediating the virus resistance or gene silencing (as proposed by Baulcombe, 1996) are:
1: The cRNA hybridises with transgene mRNA or viral RNA and the duplex becomes a target for dsRNases.
2: The cRNA hybridises with target RNA to form a duplex which can arrest translation and consequently have an indirect effect on stability (Green, 1993).
3: The duplex formed between the cRNA and viral RNA causes hybrid arrest of translation of co-factors required for viral replication.
4. The hybridization of the cRNA affects intra-molecular base pairing required for viral replication.

The current models for virus resistance or gene silencing thus involve the induction of a cytoplasmic surveillance system by either high levels of transgene transcription or by the production of aberrant single stranded mRNA. Once the system is triggered, RNA dependent RNA polymerase makes cRNA from the transgene mRNA. These cRNAs hybridise to the target RNA either directly affecting its translatability or stability, or marking the RNA for degradation.

US 5,190,131 and EP 0 467 349 A1 describe methods and means to regulate or inhibit gene expression in a cell by incorporating into or associating with the genetic material of the cell a non-native nucleic acid sequence which is transcribed to produce an mRNA which is complementary to and capable of binding to the mRNA produced by the genetic material of that cell.

EP 0 223 399 A1 describes methods to effect useful somatic changes in plants by causing the transcription in the plant cells of negative RNA strands which are substantially complementary to a target RNA strand. The target RNA strand can be a mRNA transcript created in gene expression, a viral RNA, or other RNA present in the plant cells. The negative RNA strand is complementary to at least a portion of the target RNA strand to inhibit its activity *in vivo.*

EP 0 240 208 describes a method to regulate expression of genes encoded for in plant cell genomes, achieved by integration of a gene under the transcriptional control of a promoter which is functional in the host and in which the transcribed strand of DNA is complementary to the strand of DNA that is transcribed from the endogenous gene(s) one wishes to regulate.

EP 0 647 715 A1 and US patents 5, 034,323, 5,231,020 and 5,283,184 describe methods and means for producing plants exhibiting desired phenotypic traits, by selecting transgenotes that comprise a DNA segment operably linked to a promoter, wherein transcription products of the segment are substantially homologous to corresponding transcripts of endogenous genes, particularly endogenous flavonoid biosynthetic pathway genes.

WO 93/23551 describes methods and means for the inhibition of two or more target genes, which comprise introducing into the plant a single control gene which has distinct DNA regions homologous to each of the target genes and a promoter operative in plants adapted to transcribe from such distinct regions RNA that inhibits expression of each of the target genes.

WO92/13070 describes a method for the regulation of nucleic acid translation, featuring a responsive RNA molecule which encodes a polypeptide and further includes a regulatory domain, a substrate region and a ribosome recognition sequence. This responsive RNA molecule has an inhibitor region in the regulatory domain, which regulatory domain is complementary to both a substrate region of the responsive RNA molecule and to an anti-inhibitor region of a signal nucleic acid such that, in the absence of the signal nucleic acid, the inhibitor and substrate regions form a base-paired domain the formation of which reduced the level of translation of one of the protein-coding regions in the responsive RNA molecule compared to the level of translation of that one protein-coding region observed in the presence of the signal nucleic acid.

Metzlaff *et al.,* 1997 describe a model for the RNA-mediated RNA degradation and chalcone synthase A silencing in Petunia, involving cycles of RNA-RNA pairing between complementary sequences followed by endonucleolytic RNA cleavages to describe how RNA degradation is likely to be promoted.
Fire *et al.,* 1998 describe specific genetic interference by experimental introduction of double-stranded RNA in *Caenorhabditis elegans.* The importance of these findings for functional genomics is discussed (Wagner and Sun, 1998).

Que *et al.,* 1998 describe distinct patterns of pigment suppression which are produced by allelic sense and antisense chalcone synthase transgenes in petunia flowers and have also analysed flower colour patterns in plants heterozygous for sense and antisense chalcone synthase alleles.

WO 98/05770 discloses antisense RNA with special secondary structures which may be used to inhibit gene expression.

WO 94/18337 discloses transformed plants which have increased or decreased linolenic acids as well as plants which express a linoleic acid desaturase.

US 5,850,026 discloses an endogenous oil from Brassica seeds that contains, after crushing and extracting, greater than 86% oleic acid and less than 2.5% α-linolenic acid. The oil also contains less than 7% linoleic acid. The Brassica seeds are produced by plants that contain seed-specific inhibition of microsomal oleate desaturase and microsomal linoleate desaturase gene expression, wherein the inhibition can be created by cosuppression or antisense technology.

US 5,638,637 discloses vegetable oil from rapeseeds and rapeseed producing the same, the vegetable oil having an unusually high oleic acid content of 80% to 90% by weight based on total fatty acid content.

### SUMMARY OF THE INVENTION

The present invention provides methods for reducing the phenotypic expression of a nucleic acid of interest, which is normally capable of being expressed in a eucaryotic cell, particularly for reducing the phenotypic expression of a gene, particularly a endogenous gene or a foreign transgene, integrated in the genome of a eucaryotic cell or for reducing the phenotypic expression of nucleic acid of interest which is comprised in the genome of an infecting virus, comprising the step of introducing, preferably integrating, in the nuclear genome of the eucaryotic cell, a chimeric DNA comprising a promoter, capable of being expressed in that eucaryotic cell, and optionally a DNA region involved in transcription termination and polyadenylation and in between a DNA region, which when transcribed, yields an RNA molecule with a nucleotide sequence comprising a sense nucleotide sequence of at least 10 consecutive nucleotides, particularly at least about 550 consecutive nucleotides, having between 75 and 100% sequence identity with at least part of the nucleotide sequence of the nucleic acid of interest, and an antisense nucleotide sequence including at least 10 consecutive nucleotides, having between about 75% to about 100% sequence identity with the 10 nucleotide stretch of the complement of the sense nucleotide sequence, wherein the RNA is capable of forming an artificial hairpin RNA structure with a double stranded RNA stem by base-pairing between the regions with sense and antisense nucleotide sequence such that at least the 10 consecutive nucleotides of the sense sequence basepair with the 10 consecutive nucleotides of the antisense sequence, resulting in, preferably an artificial hairpin structure. Preferably the chimeric DNA is stably integrated in the genome of the DNA.

The invention also provides a method for reducing the phenotypic expression of a nucleic acid of interest, which is normally capable of being expressed in a eucaryotic cell comprising the step of introducing a chimeric RNA molecule with a nucleotide sequence comprising a sense nucleotide sequence of at least 10 consecutive nucleotides having between 75 and 100% sequence identity with at least part of the nucleotide sequence of the nucleic acid of interest; and an antisense nucleotide sequence including at least 10 consecutive nucleotides, having between about 75% to about 100% sequence identity with 10 nt stretch of the complement of the sense nucleotide sequence; wherein the RNA is capable of forming a double stranded RNA region by base-pairing between the regions with sense and antisense nucleotide sequence such that at least the 10 consecutive nucleotides of the sense sequence basepair with the 10 consecutive nucleotides of the antisense sequence, resulting in a(n artificial) hairpin RNA structure.

The invention further provides a method for reducing the gene expression of a gene of interest in plant cells, comprising the step of introducing a first and second chimeric DNA, linked on one recombinant DNA such that both chimeric DNAs are integrated together in the nuclear genome of the transgenic plant cells; wherein the first chimeric DNA comprises a plant-expressible promoter, a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of the gene of interest and optionally a DNA region involved in transcription termination and polyadenylation functioning in plant cells. The second chimeric DNA comprises a plant-expressible promoter, a second DNA region capable of being transcribed into an antisense RNA molecule with a nucleotide sequence comprising an antisense nucleotide sequence including at least 10 consecutive nucleotides, having between about 75% to about 100% sequence identity with the complement of the at least 10 consecutive nucleotides of the sense nucleotide sequence and optionally a DNA region involved in transcription termination and polyadenylation functioning in plant cells. The sense and antisense RNA molecules are capable of forming a double stranded, duplex RNA by base-pairing between the regions which are complementary.

Also provided by the invention is a method for obtaining virus resistant organisms, particularly plants, comprising the steps of providing cells of the organism with a first and second chimeric DNA, wherein the first chimeric DNA comprises a promoter, a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of the genome of a virus, capable of infecting the plant and a DNA region involved in transcription termination and polyadenylation functioning in plant cells. The second chimeric DNA comprises a promoter, a second DNA region capable of being transcribed into an antisense RNA molecule with a nucleotide sequence comprising an antisense nucleotide sequence including at least 10 consecutive nucleotides, having between about 75% to about 100% sequence identity with the complement of at least 10 consecutive nucleotides of the sense nucleotide sequence and a DNA region involved in transcription termination and polyadenylation functioning in plant cells. The sense and antisense RNA molecules are capable of forming a double stranded RNA region by base-pairing between the regions which are complementary.

The first and second chimeric DNA are either integrated separately in the nuclear genome of the transformed plant cell or they are linked on one recombinant DNA such that both chimeric DNAs are integrated together in the nuclear genome of the transgenic plant cells.

The invention also provides a method for identifying a phenotype associated with the expression of a nucleic acid of interest in a eucaryotic cell, comprising the steps of selecting within the nucleotide sequence of interest, a target sequence of at least 10 consecutive nucleotides; designing a sense nucleotide sequence corresponding to the length of the selected target sequence and which has a sequence identity of at least about 75% to about 100% with the selected target sequence, designing an antisense nucleotide sequence which has a sequence identity of at least about 75% to about 100% with the complement of the sense nucleotide sequence and comprises a stretch of at least about 10 consecutive nucleotides with 100% sequence identity to the complement of a part of the sense nucleotide sequence. The method further comprises the steps of introducing an RNA molecule comprising both the designed sense and antisense nucleotide sequences into a suitable eucaryotic host cell comprising the nucleic acid including the nucleotide sequence with hitherto unidentified phenotype; and observing the phenotype by a suitable method.

The invention also provides a eucaryotic cell, comprising a nucleic acid of interest which is normally capable of being phenotypically expressed, further comprising a chimeric DNA molecule comprising a promoter, capable of being expressed in that eucaryotic cell, a DNA region, which when transcribed, yields an RNA molecule with a nucleotide sequence comprising a sense nucleotide sequence of at least 10 consecutive nucleotides having between 75 and 100% sequence identity with at least part of the nucleotide sequence of the nucleic acid of interest and an antisense nucleotide sequence including at least 10 consecutive nucleotides, having between about 75% to about 100% sequence identity with the complement of the at least 10 consecutive nucleotides of the sense nucleotide sequence wherein the RNA molecule is capable of forming a double stranded RNA region by base-pairing between the regions with sense and antisense nucleotide sequence such that at least said 10 consecutive nucleotides of the sense sequence basepair with said 10 consecutive nucleotides of the antisense sequence, resulting in a hairpin RNA structure, preferably an artificial hairpin structure and a DNA region involved in transcription termination and polyadenylation, wherein the phenotypic expression of the nucleic acid of interest is significantly reduced.

Also provided by the invention is a eucaryotic cell, comprising a nucleic acid of interest, which is normally capable of being phenotypically expressed, further comprising a chimeric RNA molecule with a nucleotide sequence comprising a sense nucleotide sequence of at least 10 consecutive nucleotides having between 75 and 100% sequence identity with at least part of the nucleotide sequence of the nucleic acid of interest and an antisense nucleotide sequence including at least 10 consecutive nucleotides, having between about 75% to about 100% sequence identity with the complement of the at least 10 consecutive nucleotides of the sense nucleotide sequence wherein the RNA is capable of forming a double stranded RNA region by base-pairing between the regions with sense and antisense nucleotide sequence such that at least said 10 consecutive nucleotides of the sense sequence basepair with said 10 consecutive nucleotides of the antisense sequence, resulting in an artificial hairpin RNA structure.

It is another objective of the invention to provide a eucaryotic cell, comprising a gene of interest, which is normally capable of being phenotypically expressed, further comprising a first and second chimeric DNA, linked on one recombinant DNA such that both chimeric DNAs are integrated together in the nuclear genome of that eucaryotic cell wherein the first chimeric DNA comprises the following operably linked parts a promoter capable of being expressed in the eucaryotic cell a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of the gene of interest; and a DNA region involved in transcription termination and polyadenylation; and wherein the second chimeric DNA comprises the following operably linked parts: a promoter operative in the eucaryotic cell; a second DNA region capable of being transcribed into an antisense RNA molecule with a nucleotide sequence comprising an antisense nucleotide sequence including at least 10 consecutive nucleotides, having between about 75% to about 100% sequence identity with the complement of the at least 10 consecutive nucleotides of the sense nucleotide sequence; a DNA region involved in transcription termination and polyadenylation ; wherein the sense and antisense RNA molecules are capable of forming a double stranded RNA region by base-pairing between the regions which are complementary.

It is yet another objective of the invention to provide a virus resistant plant, comprising a first and second chimeric DNA integrated in the nuclear genome of its cells, wherein the first chimeric DNA comprises a plant-expressible promoter, a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of the genome of a virus, capable of infecting the plant, and a DNA region involved in transcription termination and polyadenylation functioning in plant cells. The second chimeric DNA comprises a plant-expressible promoter, a second DNA region capable of being transcribed into an antisense RNA molecule with a nucleotide sequence comprising an antisense nucleotide sequence including at least 10 consecutive nucleotides, having between about 75% to about 100% sequence identity with the complement of the at least 10 consecutive nucleotides of the sense nucleotide sequence, and a DNA region involved in transcription termination and polyadenylation functioning in plant cells. The sense and antisense RNA molecules are capable of forming a double stranded RNA region by base-pairing between the regions which are complementary. The first and second chimeric DNA are integrated either in one locus or in different loci in the nuclear genome.

The invention also provides a method for modifying the fatty acid profile in oil, preferably increasing the oleic acid content, from a plant, preferably from oilseed rape , the method comprising the step of introducing a chimeric DNA into the cells of the plant, comprising the following operably linked parts: a). a plant-expressible promoter, preferably a seed-specific promoter; b). a DNA region, particularly with the nucleotide sequence of SEQ ID No 6, which when transcribed yields an RNA molecule comprising an RNA region capable of forming an artificial stem-loop structure, wherein one of the annealing RNA sequences of the stem-loop structure comprises a nucleotide sequence essentially similar to at least part of the nucleotide sequence of a Δ12 desaturase encoding open reading frame, and wherein the second of the annealing RNA sequences comprises a sequence essentially similar to at least part of the complement of at least part of the nucleotide sequence of the Δ12 desaturase encoding open reading frame; and optionally; c) a DNA region involved in transcription termination and polyadenylation. Plants with modified fatty acid profile, particularly with increased oleic acid content, comprising the mentioned chimeric genes are also provided by the invention. Also encompassed are oil obtained from such plants or seed.

With the foregoing and other objects, advantages and features of the invention that will become hereinafter apparent, the nature of the invention may be more clearly understood by reference to the following detailed description of the preferred embodiments of the invention and to the appended claims.

### BRIEF DESCRIPTION OF THE DRAWINGS

**Figure 1** represents schematically the different sense and antisense constructs, as well as the so-called CoP (complementary pair) constructs used for obtaining virus resistance (Fig 1 B) or for reducing the phenotypic expression of a transgenic Gus gene (Fig 1A).
**Figure 2A** represents schematically the so-called panhandle construct or CoP constructs used for reducing the phenotypic expression of a Δ12 desaturase gene in Arabidopsis (Nos Pro: nopaline synthase gene promoter; nptII neomycin phosphotransferase coding region; Nos term: nopaline syntase gene terminator; FP1: truncated seed specific napin promoter; 480 bp: 5' end of the Fad2 gene of Arabidopsis thaliana in sense orientation; 623 bp: spacer; 480 bp: 5' end of the Fad2 gene of Arabidopsis thaliana in antisense orientation.
**Figure 2B** represents schematically a common cosuppression construct for for reducing the phenotypic expression of a Δ12 desaturase gene in Arabidopsis thaliana.

### DETAILED DESCRIPTION OF THE PREFERRED EMBODIMENTS OF THE INVENTION

One of the objectives of the invention is to provide a eucaryotic cell, particularly a plant cell with an RNA molecule which comprises a hairpin structure including a determined sense part and a determined antisense part. Potentially, an RNA molecule is capable of forming several secondary structures, some of which may contain the desired hairpin. It is expected that the real secondary structure of the RNA in the cell, will have the lowest free energy. In accordance with the invention, the RNA molecule to be produced in the cell is designed in such a way that at least in its lowest free energy state, which it can assume within that cell, it will comprise the desired hairpin.

As used herein "hairpin RNA" refers to any self-annealing double stranded RNA molecule. In its simplest representation, a hairpin RNA consists of a double stranded stem made up by the annealing RNA strands, connected by a single stranded RNA loop, and is also referred to as a "pan-handle RNA". However, the term "hairpin RNA" is also intended to encompass more complicated secondary RNA structures comprising self-annealing double stranded RNA sequences, but also internal bulges and loops. The specific secondary structure adapted will be determined by the free energy of the RNA molecule, and can be predicted for different situations using appropriate software such as FOLDRNA (Zuker and Stiegler, 1981).

As used herein, "sequence identity" with regard to nucleotide sequences (DNA or RNA), refers to the number of positions with identical nucleotides divided by the number of nucleotides in the shorter of the two sequences. The alignment of the two nucleotide sequences is performed by the Wilbur and Lipmann algorithm (Wilbur and Lipmann, 1983) using a window-size of 20 nucleotides, a word length of 4 nucleotides, and a gap penalty of 4. Computer-assisted analysis and interpretation of sequence data, including sequence alignment as described above, can, e.g., be conveniently performed using the programs of the Intelligenetics™ Suite (Intelligenetics Inc., CA). Sequences are indicated as "essentially similar" when such sequence have a sequence identity of at least about 75%, particularly at least about 80 %, more particularly at least about 85%, quite particularly about 90%, especially about 95%, more especially about 100%, quite especially are identical. It is clear than when RNA sequences are said to be essentially similar or have a certain degree of sequence identity with DNA sequences, thymine (T) in the DNA sequence is considered equeal to uracil (U) in the RNA sequence.

As used herein, the term "plant-expressible promoter" means a DNA sequence which is capable of controlling (initiating) transcription in a plant cell. This includes any promoter of plant origin, but also any promoter of non-plant origin which is capable of directing transcription in a plant cell, i.e., certain promoters of viral or bacterial origin such as the CaMV35S, the subterranean clover virus promoter No 4 or No 7, or T-DNA gene promoters.

The term "expression of a gene" refers to the process wherein a DNA region which is operably linked to appropriate regulatory regions, particularly to a promoter, is transcribed into an RNA which is biologically active i.e., which is either capable of interaction with another nucleic acid or which is capable of being translated into a polypeptide or protein. A gene is said to encode an RNA when the end product of the expression of the gene is biologically active RNA, such as e.g. an antisense RNA, a ribozyme or a replicative intermediate. A gene is said to encode a protein when the end product of the expression of the gene is a protein or polypeptide.

A nucleic acid of interest is "capable of being expressed", when said nucleic acid, when introduced in a suitable host cell, particularly in a plant cell, can be transcribed (or replicated) to yield an RNA, and/or translated to yield a polypeptide or protein in that host cell.

The term "gene" means any DNA fragment comprising a DNA region (the "transcribed DNA region") that is transcribed into a RNA molecule (e.g., a mRNA) in a cell operably linked to suitable regulatory regions, e.g., a plant-expressible promoter. A gene may thus comprise several operably linked DNA fragments such as a promoter, a 5' leader sequence, a coding region, and a 3' region comprising a polyadenylation site. A plant gene endogenous to a particular plant species (endogenous plant gene) is a gene which is naturally found in that plant species or which can be introduced in that plant species by conventional breeding. A chimeric gene is any gene which is not normally found in a plant species or, alternatively, any gene in which the promoter is not associated in nature with part or all of the transcribed DNA region or with at least one other regulatory region of the gene.

As used herein, "phenotypic expression of a nucleic acid of interest" refers to any quantitative trait associated with the molecular expression of a nucleic acid in a host cell and may thus include the quantity of RNA molecules transcribed or replicated, the quantity of post-transcriptionally modified RNA molecules, the quantity of translated peptides or proteins, the activity of such peptides or proteins.

A "phenotypic trait" associated with the phenotypic expression of a nucleic acid of interest refers to any quantitative or qualitative trait, including the trait mentioned, as well as the direct or indirect effect mediated upon the cell, or the organism containing that cell, by the presence of the RNA molecules, peptide or protein, or posttranslationally modified peptide or protein. The mere presence of a nucleic acid in a host cell, is not considered a phenotypic expression or a phenotypic trait of that nucleic acid, even though it can be quantitatively or qualitatively traced. Examples of direct or indirect effects mediated on cells or organisms are, e.g., agronomically or industrial useful traits, such as resistance to a pest or disease; higher or modified oil content etc.

As used herein, "reduction of phenotypic expression" refers to the comparison of the phenotypic expression of the nucleic acid of interest to the eucaryotic cell in the presence of the RNA or chimeric genes of the invention, to the phenotypic expression of the nucleic acid of interest in the absence of the RNA or chimeric genes of the invention. The phenotypic expression in the presence of the chimeric RNA of the invention should thus be lower than the phenotypic expression in absence thereof, preferably be only about 25%, particularly only about 10%, more particularly only about 5% of the phenotypic expression in absence of the chimeric RNA, especially the phenotypic expression should be completely inhibited for all practical purposes by the presence of the chimeric RNA or the chimeric gene encoding such an RNA.

A reduction of phenotypic expression of a nucleic acid where the phenotype is a qualitative trait means that in the presence of the chimeric RNA or gene of the invention, the phenotypic trait switches to a different discrete state when compared to a situation in which such RNA or gene is absent. A reduction of phenotypic expression of a nucleic acid may thus, a.o., be measured as a reduction in transcription of (part of) that nucleic acid, a reduction in translation of (part of) that nucleic acid or a reduction in the effect the presence of the transcribed RNA(s) or translated polypeptide(s) have on the eucaryotic cell or the organism, and will ultimately lead to altered phenotypic traits. It is clear that the reduction in phenotypic expression of a nucleic acid of interest, may be accompanied by or correlated to an increase in a phenotypic trait.

As used herein "a nucleic acid of interest" or a "target nucleic acid" refers to any particular RNA molecule or DNA sequence which may be present in a eucaryotic cell, particularly a plant cell.

As used herein "comprising" is to be interpreted as specifying the presence of the stated features, integers, steps or components as referred to, but does not preclude the presence or addition of one or more features, integers, steps or components, or groups thereof. Thus, e.g., a nucleic acid or protein comprising a sequence of nucleotides or amino acids, may comprise more nucleotides or amino acids than the actually cited ones, i.e., be embedded in a larger nucleic acid or protein. A chimeric gene comprising a DNA region which is functionally or structurally defined, may comprise additional DNA regions etc.

It has been unexpectedly found by the inventors, that introduction of a chimeric gene capable of being transcribed into an RNA molecule with a nucleotide sequence comprising both the sense and antisense nucleotide sequence of a target gene, or part thereof, integrated in the nuclear genome of a plant cell, could efficiently and specifically reduce the phenotypic expression of that target gene. The reduction in phenotypic expression was more efficient and more predictable than observed when separate chimeric genes were introduced in similar cells with the target gene, encoding either sense or antisense RNA molecules.

At the same time, it has also been found that simultaneously introducing separate chimeric genes in one cell encoding RNA molecules with nucleotide sequences comprising sense and antisense respectively, resulted in extreme virus resistance, even when the chimeric genes were transcribed from weaker promoters. It is well known that gene silencing and virus resistance can be mediated by similar phenomena.

In one embodiment of the invention, a method for reducing the phenotypic expression of a nucleic acid of interest, which is normally capable of being expressed in a eucaryotic cell, particularly a plant cell, is provided, comprising the steps of introducing a chimeric DNA comprising the following operably linked parts:
a) a promoter, operative in that cell, particularly a plant-expressible promoter;
b) a DNA region capable of being transcribed into an RNA molecule with a nucleotide sequence comprising
   i. a sense nucleotide sequence of at least 10 nt, preferably 15 nt consecutive nucleotides having between 75 and 100% sequence identity with at least part of the nucleotide sequence of the nucleic acid of interest; and
   ii. an antisense nucleotide sequence including at least 10, preferably 15 nt consecutive nucleotides, having between about 75% to about 100% sequence identity with the complement of the at least 10 , preferably 15 nt consecutive nucleotides of the sense nucleotide sequence;
   wherein the RNA is capable of forming a double stranded RNA by base-pairing between the regions with sense and antisense nucleotide sequence resulting in a hairpin RNA structure; and
c) a DNA region involved in transcription termination and polyadenylation functioning in the suitable eucaryotic cells, particularly functioning in plant cells.

In a preferred embodiment of the invention, the RNA molecule transcribed from the chimeric gene, consists essentially of the hairpin RNA.

The order of the sense and antisense nucleotide sequence in the RNA molecule is thought not be critical.

Thus, in other words, the chimeric DNA has a transcribed DNA region, which when transcribed, yields an RNA molecule comprising an RNA region capable of forming an artificial stem-loop structure, wherein one of the annealing RNA sequences of the stem-loop structure comprises a sequence, essentially similar to at least part of the nucleotide sequence of the nucleic acid of interest, and wherein the second of the annealing RNA sequences comprises a sequence essentially similar to at least part of the complement of at least part of the nucleotide sequence of the nucleic acid of interest.

The RNA molecule may comprise several artificial hairpin structures, which may be designed to reduce the phenotypic expression of different nucleic acids of interest.

In one preferred embodiment, the nucleic acid of interest, whose phenotypic expression is targeted to be reduced, is a gene incorporated in the genome of a eucaryotic cell, particularly a plant cell. It will be appreciated that the means and methods of the invention can be used for the reduction of phenotypic expression of a gene which belongs to the genome of the cell as naturally occurring, (an endogenous gene), as well as for the reduction of phenotypic expression of a gene which does not belong to the genome of the cell as naturally occurring, but has been introduced in that cell (a transgene). The transgene can be introduced stably or transiently, and can be integrated into the nuclear genome of the cell, or be present on a replicating vector, such as a viral vector.

In another preferred embodiment, the nucleic acid of interest, whose phenotypic expression is targeted to be reduced is a viral nucleic acid, particularly a viral RNA molecule, capable of infecting a eucaryotic cell, particularly a plant cell. In this case, the phenotype to be reduced is the replication of the virus, and ultimately, the disease symptoms caused by the infecting virus.

Preferably, the nucleotide sequence of the target nucleic acid corresponding to the sense nucleotide sequence is part of a DNA region which is transcribed, particularly a DNA region which is transcribed and translated (in other words a coding region). It is particularly preferred that the target sequence corresponds to one or more consecutive exons, more particularly is located within a single exon of a coding region.

The length of the sense nucleotide sequence may vary from about 10 nucleotides (nt) up to a length equaling the length (in nucleotides) of the target nucleic acid. Preferably the total length of the sense nucleotide sequence is at least 10 nt, preferably 15 nt, particularly at least about 50 nt, more particularly at least about 100 nt, especially at least about 150 nt, more especially at least about 200 nt, quite especially at least about 550 nt. It is expected that there is no upper limit to the total length of the sense nucleotide sequence, other than the total length of the target nucleic acid. However for practical reason (such as e.g. stability of the chimeric genes) it is expected that the length of the sense nucleotide sequence should not exceed 5000 nt, particularly should not exceed 2500 nt and could be limited to about 1000 nt.

It will be appreciated that the longer the total length of the sense nucleotide sequence is, the less stringent the requirements for sequence identity between the total sense nucleotide sequence and the corresponding sequence in the target gene become. Preferably, the total sense nucleotide sequence should have a sequence identity of at least about 75% with the corresponding target sequence, particularly at least about 80 %, more particularly at least about 85%, quite particularly about 90%, especially about 95%, more especially about 100%, quite especially be identical to the corresponding part of the target nucleic acid. However, it is preferred that the sense nucleotide sequence always includes a sequence of about 10 consecutive nucleotides, particularly about 20 nt, more particularly about 50 nt, especially about 100 nt, quite especially about 150 nt with 100% sequence identity to the corresponding part of the target nucleic acid. Preferably, for calculating the sequence identity and designing the corresponding sense sequence, the number of gaps should be minimized, particularly for the shorter sense sequences.

The length of the antisense nucleotide sequence is largely determined by the length of the sense nucleotide sequence, and will preferably correspond to the length of the latter sequence. However, it is possible to use an antisense sequence which differs in length by about 10%. Similarly, the nucleotide sequence of the antisense region is largely determined by the nucleotide sequence of the sense region, and preferably is identical to the complement of the nucleotide sequence of the sense region. Particularly with longer antisense regions, it is however possible to use antisense sequences with lower sequence identity to the complement of the sense nucleotide sequence, preferably with at least about 75% sequence identity, more preferably with at least about 80%, particularly with at least about 85%, more particularly with at least about 90% sequence identity, especially with at least about 95% sequence to the complement of the sense nucleotide sequence. Nevertheless, it is preferred that the antisense nucleotide sequences always includes a sequence of about 10 , preferably 15 consecutive nucleotides, particularly about 20 nt, more particularly about 50 nt, especially about 100 nt, quite especially about 150 nt with 100% sequence identity to the complement of a corresponding part of the sense nucleotide sequence. It is clear that the length of the stretch of the consecutive nucleotides with 100% sequence identity to the complement of the sense nucleotide sequence cannot be longer than the sense nucleotide sequence itself. Again, preferably the number of gaps should be minimized, particularly for the shorter antisense sequences. Further, it is also preferred that the antisense sequence has between about 75% to 100% sequence identity with the complement of the target sequence.

The RNA molecule resulting from the transcription of the chimeric DNA may comprise a spacer nucleotide sequence located between the sense and antisense nucleotide sequence. In the absence of such a spacer sequence, the RNA molecule will still be able to form a double-stranded RNA, particularly if the sense and antisense nucleotide sequence are larger than about 10 nucleotides and part of the sense and/or antisense nucleotide sequence will be used to form the loop allowing the base-pairing between the regions with sense and antisense nucleotide sequence and formation of a double stranded RNA. It is expected that there are no length limits or sequence requirements associated with the spacer region, as long as these parameters do not interfere with the capability of the RNA regions with the sense and antisense nucleotide sequence to form a double stranded RNA. In a preferred embodiment, the spacer region varies in length from 4 to about 200 bp, but as previously mentioned, it may be absent.

In a preferred embodiment, the hairpin RNA formed by the sense and antisense region and if appropriate the spacer region, is an artificial hairpin RNA. By "artificial hairpin RNA" or "artificial stem-loop RNA structure", is meant that such hairpin RNA is not naturally occurring in nature, because the sense and antisense regions as defined are not naturally occurring simultaneously in one RNA molecule, or the sense and antisense regions are separated by a spacer region which is heterologous with respect to the target gene, particularly, the nucleotide sequence of the spacer has a sequence identity of less than 75% with the nucleotide sequence of the target sequence, at the corresponding location 5' or 3' of the endpoints of the sense nucleotide sequence. A hairpin RNA can also be indicated as artificial, if it is not comprised within the RNA molecule it is normally associated with. It is conceivable to use in accordance with the invention a chimeric DNA whose transcription results in a hairpin RNA structure with a naturally occurring nucleotide sequence (which otherwise meets the limits as set forth in this specification) provided this hairpin RNA is devoid of the surrounding RNA sequences (not involved in the hairpin structure formation).

Although it is preferred that the RNA molecule comprising the hairpin RNA does not further comprise an intron sequence, it is clear that the chimeric DNA genes encoding such RNAs may comprise in their transcribed region one or more introns.

In fact, the inventors have unexpectedly found that inclusion of an intron sequence in the chimeric DNA genes encoding an RNA molecule comprising the hairpin RNA, preferably in the spacer region, and preferably in sense orientation, enhances the efficiency of reduction of expression of the target nucleic acid. The enhancement in efficiency may be expressed as an increase in the frequency of plants wherein silencing occurs or as an increase in the level of reduction of the phenotypic trait. In a particularly preferred embodiment, the intron is essentially identical in sequence to the Flaveria trinervia pyruvate orthophosphate dikinase 2 intron 2, more particularly, it comprises the sequence of SEQ ID No 7.

It has been observed that contrary to methods using either antisense or sense nucleotide sequences alone to reduce the phenotypic expression of a target nucleic acid (which generally depend on the dosage of sense or antisense molecule, and thus the chimeric genes encoding the sense and antisense molecules need to be under the control of relatively strong promoters) the method of the current invention does not rely on the presence of such strong promoter regions to drive the transcriptional production of the RNA comprising both the sense and antisense region. In other words, a whole range of promoters, particularly plant expressible promoters, is available to direct the transcription of the chimeric genes of the invention. These include, but are not limited to strong promoters such as CaMV35S promoters (e.g., Harpster et *al.,* 1988). In the light of the existence of variant forms of the CaMV35S promoter, as known by the skilled artisan, the object of the invention can equally be achieved by employing these alternative CaMV35S promoters and variants. It is also clear that other plant-expressible promoters, particularly constitutive promoters, such as the opine synthase promoters of the *Agrobacterium* Ti- or Ri-plasmids, particularly a nopaline synthase promoter, or subterranean clover virus promoters can be used to obtain similar effects. Also contemplated by the invention are chimeric genes to reduce the phenotypic expression of a nucleic acid in a cell, which are under the control of single subunit bacteriophage RNA polymerase specific promoters, such as a T7 or a T3 specific promoter, provided that the host cells also comprise the corresponding RNA polymerase in an active form.

It is a further object of the invention, to provide methods for reducing the phenotypic expression of a nucleic acid in specific cells, particularly specific plant cells by placing the chimeric genes of the invention under control of tissue-specific or organ-specific promoters. Such tissue-specific or organ-specific promoters are well known in the art and include but are not limited to seed-specific promoters (e.g., WO89/03887), organ-primordia specific promoters (An *et al.,* 1996), stem-specific promoters (Keller *et al.,* 1988), leaf specific promoters (Hudspeth *et al.* ,1989), mesophyl-specific promoters (such as the light-inducible Rubisco promoters), root-specific promoters (Keller *et al*.,1989), tuber-specific promoters (Keil *et al.,* 1989), vascular tissue specific promoters ( Peleman *et al.,* 1989 ), stamen-selective promoters ( WO 89/10396, WO 92/13956), dehiscence zone specific promoters ( WO 97/13865) and the like.

In another embodiment of the invention, the expression of a chimeric gene to reduce the phenotypic expression of a target nucleic acid can be controlled at will by the application of an appropriate chemical inducer, by operably linking the transcribed DNA region of the chimeric genes of the invention to a promoter whose expression is induced by a chemical compound, such as the promoter of the gene disclosed in European Patent publication ("EP") 0332104, or the promoter of the gene disclosed in WO 90/08826.

It has been found that a similar reduction in phenotypic expression of a nucleic acid of interest in a eucaryotic cell, particularly in a plant cell, can be obtained by providing the sense and antisense RNA encoding ranscribable DNA regions as separate transgenes, preferably located in one locus, particularly as one allele.

Thus, in another embodiment of the invention a method for reducing the phenotypic expression of a nucleic acid which is normally capable of being expressed in a eucaryotic cell, particularly a plant cell, is provided, comprising the steps of introducing a first and second chimeric DNA, linked on one recombinant DNA such that both chimeric DNAs are integrated together in the nuclear genome of the transgenic cells;
wherein the first chimeric DNA comprises the following operably linked parts:
a) a promoter, operative in the cell, particularly a plant-expressible promoter;
b) a DNA region capable of being transcribed into a sense RNA molecule with a nucleotide sequence comprising a sense nucleotide sequence of at least 10, preferably 15 consecutive nucleotides having between 75 and 100% sequence identity with at least part of the nucleotide sequence of the nucleic acid of interest; and
c) a DNA region involved in transcription termination and polyadenylation functioning in the corresponding eucaryotic cell; and
wherein the second chimeric DNA comprises the following operably linked parts:
a) a promoter, operative in the cell, particularly a plant-expressible promoter;
b) a DNA region capable of being transcribed into an antisense RNA molecule with a nucleotide sequence comprising an antisense nucleotide sequence including at least 10 , preferably 15 consecutive nucleotides, having between about 75% to about 100% sequence identity with the complement of the at least 10 , preferably 15 consecutive nucleotides of the sense nucleotide sequence;
c) a DNA region involved in transcription termination and polyadenylation functioning in the corresponding eucaryotic cell;
wherein the sense and antisense RNA are capable of forming a double stranded RNA by base-pairing between the regions which are complementary.

Preferred embodiments for the different structural and functional characteristics, such as length and sequence of sense, antisense and spacer regions, of this method are as described elsewhere in this specification.

The RNA molecule, comprising the sense and antisense nucleotide sequences capable of forming a hairpin structure, which are produced by the transcription of the chimeric genes, can also be introduced directly in a plant cell to reduce the phenotypic expression of the target nucleic acid, particularly to reduce the phenotypic expression of a targeted endogenous gene, or a targeted transgene. Such RNA molecules could be produced e.g. by
1. cloning the DNA region capable of being transcribed into an RNA molecule with a nucleotide sequence comprising a sense nucleotide sequence of at least 10 consecutive nucleotides having between 75 and 100% sequence identity with at least part of the nucleotide sequence of the nucleic acid of interest and an antisense nucleotide sequence including at least 10 consecutive nucleotides, having between about 75% to about 100% sequence identity with the complement of the at least 10 consecutive nucleotides of the sense nucleotide sequence, whereby the RNA is capable of forming a double stranded RNA by base-pairing between the regions with sense and antisense nucleotide sequence resulting in a hairpin RNA structure, under control of a promoter suitable for recognition by a DNA-dependent RNA polymerase in an *in vitro* transcription reaction, such as but not limited to a T7-polymerase specific promoter;
2. performing an *in vitro* transcription reaction by adding *inter alia* the suitable DNA -dependent RNA polymerase as well as the required reagents to generate the RNA molecules; and
3. isolating the RNA molecules.

*In vitro* transcription methods as well as other methods for *in vitro* RNA production are well known in the art and commercial kits are available.
Methods for direct introduction of RNA in plant cells are also available to the skilled person and include but are not limited to electroporation, microinjection and the like.

The chimeric gene(s) for reduction of the phenotypic expression of a target nucleic acid of interest in a cell, may be introduced either transiently, or may be stably integrated in the nuclear genome of the cell. In one embodiment, the chimeric gene is located on a viral vector, which is capable of replicating in the eucaryotic cell, particularly the plant cell (see e.g., WO 95/34668 and WO 93/03161).

The recombinant DNA comprising the chimeric gene to reduce the phenotypic expression of a nucleic acid of interest in a host cell, may be accompanied by a chimeric marker gene, particularly when the stable integration of the transgene in the genome of the host cell is envisioned. The chimeric marker gene can comprise a marker DNA that is operably linked at its 5' end to a promoter, functioning in the host cell of interest, particularly a plant-expressible promoter, preferably a constitutive promoter, such as the CaMV 35S promoter, or a light inducible promoter such as the promoter of the gene encoding the small subunit of Rubisco; and operably linked at its 3' end to suitable plant transcription 3' end formation and polyadenylation signals. It is expected that the choice of the marker DNA is not critical, and any suitable marker DNA can be used. For example, a marker DNA can encode a protein that provides a distinguishable colour to the transformed plant cell, such as the A1 gene (Meyer *et al.,* 1987), can provide herbicide resistance to the transformed plant cell, such as the *bar* gene, encoding resistance to phosphinothricin (EP 0,242,246), or can provide antibiotic resistance to the transformed cells, such as the *aac(6')* gene, encoding resistance to gentamycin (WO94/01560).

A recombinant DNA comprising a chimeric gene to reduce the phenotypic expression of a gene of interest, can be stably incorporated in the nuclear genome of a cell of a plant. Gene transfer can be carried out with a vector that is a disarmed Ti-plasmid, comprising a chimeric gene of the invention, and carried by *Agrobacterium.* This transformation can be carried out using the procedures described, for example, in EP 0 116 718.

Alternatively, any type of vector can be used to transform the plant cell, applying methods such as direct gene transfer (as described, for example, in EP 0 233 247), pollen-mediated transformation (as described, for example, in EP 0 270 356, WO85/01856 and US 4,684,611), plant RNA virus-mediated transformation (as described, for example, in EP 0 067 553 and US 4,407,956), liposome-mediated transformation (as described, for example, in US 4,536,475), and the like.

Other methods, such as microprojectile bombardment as described for corn by Fromm *et al.* (1990) and Gordon-Kamm *et al.* (1990), are suitable as well. Cells of monocotyledonous plants, such as the major cereals, can also be transformed using wounded and/or enzyme-degraded compact embryogenic tissue capable of forming compact embryogenic callus, or wounded and/or degraded immature embryos as described in WO92/09696. The resulting transformed plant cell can then be used to regenerate a transformed plant in a conventional manner.

The obtained transformed plant can be used in a conventional breeding scheme to produce more transformed plants with the same characteristics or to introduce the chimeric gene for reduction of the phenotypic expression of a nucleic acid of interest of the invention in other varieties of the same or related plant species, or in hybrid plants. Seeds obtained from the transformed plants contain the chimeric genes of the invention as a stable genomic insert.

It is a further object of the invention to provide eucaryotic cells, preferably plant cells and organisms (preferably plants) comprising the chimeric genes for the reduction of the phenotypic expression of a target nucleic acid as described in the invention.

It is a yet a further object of the invention to provide plant cells, comprising a nucleic acid of interest, which is normally capable of being expressed phenotypically, further comprising an RNA molecule with a nucleotide sequence which includes:
i. a sense nucleotide sequence of at least 10 consecutive nucleotides having between 75 and 100% sequence identity with at least part of the nucleotide sequence of the nucleic acid of interest; and
ii. an antisense nucleotide sequence including at least 10 consecutive nucleotides, having between about 75% to about 100% sequence identity with the complement of the at least 10 consecutive nucleotides of the sense nucleotide sequence and capable of forming a double stranded RNA by association with the sense nucleotide sequence;
wherein the phenotypic expression of the nucleotide acid of interest is significantly reduced by the presence of the RNA molecule, when compared to the phenotypic expression of the nucleic acid of interest in the absence of the RNA molecule.
The RNA molecule may be encoded by chimeric DNA. Preferred embodiments for the sense and antisense nucleotide sequence, particularly concerning length and sequence, are as mentioned elsewhere in this specification.

It will be appreciated that the methods and means described in the specification can also be applied in High Throughput Screening (HTS) methods, for the identification or confirmation of phenotypes associated with the expression of a nucleic acid sequence with hitherto unidentified function in a eucaryotic cell, particularly in a plant cell.
Such a method comprises the steps of
1. selecting a target sequence within the nucleic acid sequence of interest with unidentified or non-confirmed function/phenotype when expressed. Preferably, if the nucleic acid has putative open reading frames, the target sequence should comprise at least part of one of these open reading frames. The length of the target nucleotide sequence may vary from about 10 nucleotides up to a length equalling the length (in nucleotides) of the nucleic acid of interest with unidentified function.
2. designing an RNA molecule comprising sense nucleotide sequence and antisense nucleotide sequence in accordance with the invention.
3. introducing the RNA molecule comprising both the sense and antisense nucleotide sequences designed on the basis of the target sequence, into a suited host cell, particularly a plant cell, comprising the nucleic acid with the nucleotide sequence with hitherto unidentified phenotype. The RNA can either be introduced directly, or can be introduced by means of a chimeric DNA comprising a promoter operative in the host cell of interest, particularly a plant-expressible promoter, and a DNA region functioning as a suitable 3' end formation and polyadenylation signal (terminator) functioning in the host cell, with in-between a DNA region which can be transcribed to yield the RNA molecule comprising the sense and antisense nucleotide sequence. The chimeric DNA can either be introduced transiently or
   integrated in the nuclear genome. The chimeric DNA can also be provided on a viral vector (see, e.g., WO 95/34668 and WO 93/03161)
4. observing the phenotype by a suitable method. Depending on the phenotype expected, it may be sufficient to observe or measure the phenotype in a single cell, but it may also be required to culture the cells to obtain an (organized) multicellular level, or even to regenerate a transgenic organism, particularly a transgenic plant.

In its most straightforward embodiment, the RNA molecule comprising both the sense and antisense nucleotide sequences to at least part of a nucleic acid of interest, suitable for the methods of the invention, can be obtained by cloning two copies of a DNA region with the selected target sequence in inverted repeat orientation (preferably separated by a short DNA region which does not contain a transcription termination signal, and encodes the spacer sequence) under a suitable promoter. This chimeric DNA is then either used as template DNA in an *in vitro* transcription method to generate the RNA molecule, which is introduced in the host cell, or the chimeric DNA itself is introduced in the host cell.

The methods and means of the invention can thus be used to reduce phenotypic expression of a nucleic acid in a eucaryotic cell or organism, particularly a plant cell or plant, for obtaining shatter resistance (WO 97/13865), for obtaining modified flower colour patterns (EP 522 880, US 5,231,020), for obtaining nematode resistant plants (WO 92/21757, WO 93/10251, WO 94/17194), for delaying fruit ripening (WO 91/16440, WO 91/05865, WO 91/16426, WO 92/17596, WO 93/07275, WO 92/04456, US 5,545,366), for obtaining male sterility (WO 94/29465, WO89/10396, WO 92/18625), for reducing the presence of unwanted (secondary) metabolites in organisms, such as glucosinolates (WO97/16559) or chlorophyll content (EP 779 364) in plants , for modifying the profile of metabolites synthesized in a eucaryotic cell or organisms by metabolic engineering e.g. by reducing the expression of particular genes involved in carbohydrate metabolism (WO 92/11375, WO 92/11376, US 5, 365, 016, WO 95/07355) or lipid biosynthesis (WO 94/18337, US 5, 530, 192), for delaying senescence (WO 95/07993), for altering lignification in plants (WO 93/05159, WO 93/05160), for altering the fibre quality in cotton (US 5, 597, 718), for increasing bruising resistance in potatoes by reducing polyphenoloxidase (WO 94/03607), etc.

The methods of the invention will lead to better results and/or higher efficiencies when compared to the methods using conventional sense or antisense nucleotide sequences and it is believed that other sequence-specific mechanisms regulating the phenotypic expression of target nucleic acids might be involved and/or triggered by the presence of the double-stranded RNA molecules described in this specification.

A particular application for reduction of the phenotypic expression of a transgene in a plant cell, *inter alia,* by antisense or sense methods, has been described for the restoration of male fertility, the latter being obtained by introduction of a transgene comprising a male sterility DNA (WO 94/09143, WO 91/02069). The nucleic acid of interest is specifically the male sterility DNA. Again, the processes and products described in this invention can be applied to these methods in order to arrive at a more efficient restoration of male fertility.

The methods and means of the invention, particularly those involving RNA molecules comprising a hairpin RNA and the encoding chimeric genes, have proven to be particularly suited for the modification of the composition of oil content in plants, particularly in seeds. Particularly preferred plants are crop plants used for oil production such as but not limited to oilseed rape (Brassica juncea, napus, rapa, oleracea, campestris), corn, cotton, groundnut, sunflower, castor beans, flax, coconut, linseed, soybean. Preferred target genes are the desaturase genes, particularly Δ12 desaturase encoding genes such as those encoded by the Fad2 genes, especially the genes whose nucleotide sequence can be found in the Genbank Database under accession number AF123460 (from Brassica carinata), AF12042841 (Brassica rapa), L26296 (Arabidopsis thaliana) or A65102 (Corylus avellana). It is clear that it is well within the reach of the person skilled in the art to obtain genes homologous to the disclosed fad2 genes from other species e.g. by hybridization and/or PCR techniques.

Preferred embodiments for the configuration of sense and antisense nucleotide sequences, particularly concerning length and sequence are as mentioned elsewhere in this specification. Also, preferred embodiments for chimeric genes encoding hairpin containing RNA molecules, particularly concerning promoter elements are as described elsewhere in the specification. For this application, it is particularly preferred that the promoters are seed-specific promoters.

In a preferred embodiment, the artificial hairpin RNA comprising RNA molecule thus comprises part of a Δ12 desaturase encoding ORF in sense orientation and a similar part in antisense orientation, preferably separated by a spacer sequence. In a particularly preferred embodiment the artificial hairpin RNA (or its encoding chimeric gene) comprises the nucleotide sequence of SEQ ID No 6 or a similarly constructed nucleotide sequence based on the aforementioned Brassica fad2 genes.

Preferably the chimeric gene encoding the artificial hairpin RNA is transcribed under control of a seed-specific promoter, particularly under control of the FPI promoter as described elsewhere in this application.

A reduction of the expression of Δ12 desaturase gene in oil containing plants leads to increase in oleic acid and a concomitant decrease in linolenic acid and linoleic acid. A higher frequency of plants with oil wherein the increase in oleic acid and concomitant decrease in linolenic and linoleic acid is significant is found using the means and methods of the invention than in transgenic plants harbouring common cosuppression constructs. Moreover the absolute levels of increase, respectively decrease are higher respectively lower than in transgenic plants harbouring common cosuppression constructs.

Using the means and methods of the invention, it is thus possible to obtain plants and seeds, comprising an oil of which the composition after crushing and extracting has an increased oleic acid content (expressed as a percentage of the total fatty acid composition), particularly a three fold increase, when compared with control plants.

It is expected that using the methods and means of the invention, transgenic Brassica plant can be obtained, whose seeds comprise an oil wherein the oleic acid content exceeds 90% of the total fatty acid contents.

The methods and means of the invention will be especially suited for obtaining virus resistance, particularly extreme virus resistance, in eucaryotic cells or organisms, particularly in plant cells and plants. A non-limiting list of viruses for plants against which resistance can be obtained, is represented in table I.

The methods and means of the invention further allow the use of viral genes, hitherto unused for obtaining virus resistant plants in addition to the commonly used coat protein genes or replicase genes. Such different viral genes include protease encoded genes (Pro) genome linked protein (Vpg) encoding genes, cytoplasmic inclusion body encoding genes (CI) as target nucleic acid sequences for obtaining virus resistant plants.

It is clear that the invention will be especially suited for the reduction of phenotypic expression of genes belonging to multigene families.

It is also clear that the methods and means of the invention are suited for the reduction of the phenotypic expression of a nucleic acid in all plant cells of all plants, whether they are monocotyledonous or dicotyledonous plants, particularly crop plants such as but not limited to corn, rice, wheat, barley, sugarcane, cotton, oilseed rape, soybean, vegetables (including chicory, brassica vegetables, lettuce, tomato), tobacco, potato, sugarbeet but also plants used in horticulture, floriculture or forestry. The means and methods of the invention will be particularly suited for plants which have complex genomes, such as polyploid plants.

It is expected that the chimeric RNA molecules produced by transcription of the chimeric genes described herein, can spread systemically throughout a plant, and thus it is possible to reduce the phenotypic expression of a nucleic acid in cells of a non-transgenic scion of a plant grafted onto a transgenic stock comprising the chimeric genes of the invention (or vice versa) a method which may be important in horticulture, viticulture or in fruit production.

The following non-limiting Examples describe the construction of chimeric genes for the reduction of the phenotypic expression of a nucleic acid of interest in a eucaryotic cell and the use of such genes. Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK.

Throughout the description and Examples, reference is made to the following sequences:

| | |
|---|---|
| SEQ ID N° 1: | sequence of the Potato virus Y fragment of the Nia gene used for the construction of various sense and antisense constructs, for obtaining virus resistance. |
| SEQ ID N° 2: | sequence of the coding region of the Gusd CoP construct of Example 1. |
| SEQ ID N° 3 | sequence of a modified 5' untranslated region (5'UTR) from Johnsongrass mosaic virus |
| SEQ ID N° 4 | sequence of the Subterranean clover virus promoter No 4 with S7 enhancer. |
| SEQ ID N° 5 | sequence of the Subterranean clover virus double enhancer promoter No 4. |
| SEQ ID N° 6 | sequence of the CoP construct for the Δ12desaturase gene expression inhibition. |
| SEQ ID N° 7 | sequence of the Flaveria trinervia pyruvate orthophosphate dikinase intron 2 |

The following free text is included in the sequence listing :
<223> fragment of the NIa ORF
<223> Description of Artificial Sequence:coding region of the Gusd CoP construct
<223> deficient Gus coding region
<223> antisense to the 5' end of the Gus coding region
<223> Description of Artificial Sequence:5'UTR of Johnson mosaic virus
<223> Description of Artificial Sequence:Subterannean clover virus S4 promoter with S7 enhancer
<223> Description of Artificial Sequence: subterranean clover virus promoter S4 with S4 enhancer
<223> Description of Artificial Sequence: coding sequence of the desaturase CoP construct
<223> corresponding to the 5' end of the delta12-desaturase (fad2) coding region, in sense orientation
<223> corresponding to the 5' end of the delta12-desaturase (fad2) coding region, in anti sense orientation
<223> Description of Artificial Sequence: intron 2 of the Flaveria trinervia puryvate orthophosphate dikinase

Embodiments of the invention may include the features of the following enumerated items (1) to (38):
1. A method for reducing the phenotypic expression of a nucleic acid of interest, which is normally capable of being expressed in a eucaryotic cell, comprising the step of introducing a chimeric DNA comprising the following operably linked parts:
   a) a promoter, operative in said eucaryotic cell;
   b) DNA region, which when transcribed, yields an RNA molecule comprising an RNA region capable of forming an artificial stem-loop structure, wherein one of the annealing RNA sequences of the stem-loop structure comprises a sequence, essentially similar to at least part of the nucleotide sequence of said nucleic acid of interest, and wherein the second of said annealing RNA sequences comprises a sequence essentially similar to at least part of the complement of at least part of said nucleotide sequence of said nucleic acid of interest; and optionally
   c) a DNA region involved in transcription termination and polyadenylation.
2. A method for reducing the phenotypic expression of a nucleic acid of interest, which is normally capable of being expressed in a eucaryotic cell, comprising the step of introducing a chimeric DNA comprising the following operably linked parts:
   a) a promoter, operative in said eucaryotic cell;
   b) a DNA region, which when transcribed, yields an RNA molecule with a nucleotide sequence comprising
      i. a sense nucleotide sequence of at least 10 consecutive nucleotides having between 75 and 100% sequence identity with at least part of the nucleotide sequence of said nucleic acid of interest; and
      ii. an antisense nucleotide sequence including at least 10 consecutive nucleotides, having between about 75% to about 100% sequence identity with the complement of said at least 10 consecutive nucleotides of said sense nucleotide sequence;
      wherein the RNA is capable of forming an artificial hairpin RNA structure with a double stranded RNA stem by base-pairing between the regions with sense and antisense nucleotide sequence such that at least said 10 consecutive nucleotides of the sense sequence basepair with said 10 consecutive nucleotides of the antisense sequence; and optionally
   c) a DNA region involved in transcription termination and polyadenylation.
3. The method of item 2, wherein said RNA molecule further comprises a spacer nucleotide sequence located between said sense and said antisense nucleotide sequence.
4. The method of item 2, wherein said sense nucleotide sequence comprises at least about 550 consecutive nucleotides having between 75% and 100% sequence identity with at least part of the nucleotide sequence of said nucleic acid.
5. The method of item 2, wherein said nucleic acid of interest is a gene integrated in the genome of said eucaryotic cell.
6. The method of item 5, wherein said gene is an endogenous gene
7. The method of item 5, wherein said gene is a foreign transgene.
8. The method of item 2, wherein said chimeric DNA is stably integrated in the genome of the DNA.
9. The method of item 2, wherein said nucleic acid of interest is comprised in the genome of an infecting virus.
10. The method of item 9, wherein said infecting virus is an RNA virus.
11. The method according to any one of item 1, wherein said eucaryotic cell is a plant cell.
12. The method of item 11, wherein said plant cell is comprised within a plant.
13. A method for reducing the phenotypic expression of a nucleic acid of interest, which is normally capable of being expressed in a eucaryotic cell comprising the step of introducing a chimeric RNA molecule comprising at least one RNA region with a nucleotide sequence comprising
   i. a sense nucleotide sequence of at least 10 consecutive nucleotides having between 75 and 100% sequence identity with at least part of the nucleotide sequence of the nucleic acid of interest; and
   ii. an antisense nucleotide sequence including at least 10 consecutive nucleotides, having between about 75% to about 100% sequence identity with the complement of said at least 10 consecutive nucleotides of said sense nucleotide sequence;
   wherein said RNA region is capable of forming an artificial hairpin RNA structure with a double stranded RNA stem by base-pairing between the regions with sense and antisense nucleotide sequence such that at least said 10 consecutive nucleotides of the sense sequence basepair with said 10 consecutive nucleotides of the antisense sequence.
14. A method for reducing the gene expression of a gene of interest in plant cells, said method comprising the step of introducing a first and second chimeric DNA, linked on one recombinant DNA such that both chimeric DNAs are integrated together in the nuclear genome of the transgenic plant cells; wherein said first chimeric DNA comprises the following operably linked parts:
   a) a plant-expressible promoter;
   b) a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of said gene of interest; and optionally
   c) a DNA region involved in transcription termination and polyadenylation functioning in plant cells; and
   wherein said second chimeric DNA comprises the following operably linked parts:
   a) a plant-expressible promoter;
   b) a second DNA region capable of being transcribed into an antisense RNA molecule with a nucleotide sequence comprising an antisense nucleotide sequence including at least 10 consecutive nucleotides, having between about 75% to about 100% sequence identity with the complement of said at least 10 consecutive nucleotides of said sense nucleotide sequence; an optionally
   c) a DNA region involved in transcription termination and polyadenylation functioning in plant cells;
   wherein said sense and antisense RNA molecules are capable of forming a double stranded RNA by base-pairing between the regions which are complementary.
15. A method for obtaining a virus resistant eucaryotic organism, said method comprising the steps of :
   1) providing the cells of said organism with a first and second chimeric DNA wherein said first chimeric DNA comprises the following operably linked parts:
      a) a promoter operative in said cells;
      b) a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of the genome of a virus, capable of infecting said organism; and optionally
      c) a DNA region involved in transcription termination and polyadenylation functioning in said cells; and
      wherein said second chimeric DNA comprises the following operably linked parts:
      a) a promoter operative in said cells;
      b) a second DNA region capable of being transcribed into an antisense RNA molecule with a nucleotide sequence comprising an antisense nucleotide sequence including at least 10 consecutive nucleotides, having between about 75% to about 100% sequence identity with the complement of said at least 10 consecutive nucleotides of said sense nucleotide sequence; and optionally
      c) a DNA region involved in transcription termination and polyadenylation functioning in said cells; and
      wherein said sense and antisense RNA are capable of forming a double stranded RNA by base-pairing between the regions which are complementary.
16. The method according to item 15, wherein said organism is a plant
17. The method of item 16, wherein said cells of said plants are provided with said first and second chimeric DNA by crossing parent plants comprising either said first or said second chimeric DNA.
18. The method of item 16, wherein said cells of said plants are provided with said first and second chimeric DNA by transforming a plant cell with said first and second chimeric DNA, and regenerating a plant from said transformed plant cell.
19. The method of item 16, wherein said first and second chimeric DNA are integrated separately in said nuclear genome of said plant cell.
20. The method of item 16, wherein said first and second chimeric DNA are linked on one recombinant DNA such that both chimeric DNAs are integrated together in the nuclear genome of the transgenic plant cells.
21. A method for identifying a phenotype associated with the expression of a nucleic acid of interest in a eucaryotic cell, said method comprising
   a. selecting within said nucleotide sequence of interest, a target sequence of at least 10 consecutive nucleotides;
   b. designing a sense nucleotide sequence corresponding to the length of the selected target sequence and which has a sequence identity of at least about 75% to about 100% with said selected target sequence;
   c. designing an antisense nucleotide sequence which:
      i) has a sequence identity of at least about 75% to about 100% with the complement of said at least 10 consecutive nucleotides of said sense nucleotide sequence;
      ii) comprises a stretch of at least about 10 consecutive nucleotides with 100% sequence identity to the complement of a part of said sense nucleotide sequence;
   d. introducing an RNA molecule comprising both said sense and antisense nucleotide sequences into a suitable eucaryotic host cell comprising the nucleic acid including the nucleotide sequence with hitherto unidentified phenotype; and
   e. observing the phenotype by a suitable method.
22. A eucaryotic cell, comprising a nucleic acid of interest, which is normally capable of being phenotypically expressed, further comprising a chimeric DNA molecule comprising the following operably linked parts:
   a) a promoter, operative in said eucaryotic cell;
   b) a DNA region, which when transcribed, yields an RNA molecule with at least one RNA region with a nucleotide sequence comprising
      i. a sense nucleotide sequence of at least 10 consecutive nucleotides having between 75 and 100% sequence identity with at least part of the nucleotide sequence of the nucleic acid of interest; and
      ii. an antisense nucleotide sequence including at least 10 consecutive nucleotides, having between about 75% to about 100% sequence identity with the complement of said at least 10 consecutive nucleotides of said sense nucleotide sequence;
      wherein the RNA is capable of forming an artificial hairpin RNA structure with a double stranded RNA stem by base-pairing between the regions with sense and antisense nucleotide sequence; and optionally
   c) a DNA region involved in transcription termination and polyadenylation. wherein the phenotypic expression of said nucleic acid of interest is significantly reduced.
23. A eucaryotic cell, comprising a nucleic acid of interest, which is normally capable of being phenotypically expressed, further comprising a chimeric RNA molecule comprising at least one RNA region with a nucleotide sequence comprising
   i. a sense nucleotide sequence of at least 10 consecutive nucleotides having between 75 and 100% sequence identity with at least part of the nucleotide sequence of the nucleic acid of interest; and
   ii. an antisense nucleotide sequence including at least 10 consecutive nucleotides, having between about 75% to about 100% sequence identity with the complement of said at least 10 consecutive nucleotides of said sense nucleotide sequence;
   wherein said RNA is capable of forming an artifical hairpin RNA with a double stranded RNA region by base-pairing between the regions with sense and antisense nucleotide sequence such that at least said 10 consecutive nucleotides of the sense sequence basepair with said 10 consecutive nucleotides of the antisense sequence.
24. A eucaryotic cell, comprising a gene of interest, which is normally capable of being phenotypically expressed, further comprising a first and second chimeric DNA, linked on one recombinant DNA such that both chimeric DNAs are integrated together in the nuclear genome of said eucaryotic cell;
   wherein said first chimeric DNA comprises the following operably linked parts:
   a) a promoter operative in said eucaryotic cell
   b) a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of said gene of interest; and optionally
   c) a DNA region involved in transcription termination and polyadenylation; and
   wherein said second chimeric DNA comprises the following operably linked parts:
   a) a promoter operative in said eucaryotic cell;
   b) a second DNA region capable of being transcribed into an antisense RNA molecule with a nucleotide sequence comprising an antisense nucleotide sequence including at least 10 consecutive nucleotides, having between about 75% to about 100% sequence identity with the complement of said at least 10 consecutive nucleotides of said sense nucleotide sequence; and optionally
   c) a DNA region involved in transcription termination and polyadenylation;
   wherein said sense and antisense RNA molecules are capable of forming a double stranded RNA region by base-pairing between the regions which are complementary.
25. The eucaryotic cell of any one of item 22, which is a plant cell.
26. A plant comprising the plant cell of item 25.
27. A virus resistant plant, comprising a first and second chimeric DNA integrated in the nuclear genome its cells , wherein said first chimeric DNA comprises the following operably linked parts:
   a) a plant-expressible promoter;
   b) a first DNA region capable of being transcribed into a sense RNA molecule with a nucleotide sequence comprising a sense nucleotide sequence of at least 10 consecutive nucleotides having between 75 and 100% sequence identity with at least part of the nucleotide sequence of the genome of a virus, capable of infecting said plant; and optionally
   c) a DNA region involved in transcription termination and polyadenylation functioning in plant cells; and
   wherein said second chimeric DNA comprises the following operably linked parts:
   a) a plant-expressible promoter;
   b) a second DNA region capable of being transcribed into an antisense RNA molecule with a nucleotide sequence comprising an antisense nucleotide sequence including at least 10 consecutive nucleotides, having between about 75% to about 100% sequence identity with the complement of said at least 10 consecutive nucleotides of said sense nucleotide sequence;
   c) optionally, a DNA region involved in transcription termination and polyadenylation functioning in plant cells; and
   wherein said sense and antisense RNA are capable of forming a double stranded RNA region by base-pairing between the regions which are complementary.
28. The plant of item 27, wherein said first and second chimeric DNA are integrated in one locus in the nuclear genome.
29. The plant of item 27, wherein said first and second chimeric DNA are integrated in different loci in the nuclear genome.
30. A method for modifying the fatty acid profile in oil from a plant, said method comprising the step of introducing a chimeric DNA into the cells of said plant, said chimeric DNA comprising the following operably linked parts:
   a). a plant-expressible promoter
   b). a DNA region, which when transcribed yields an RNA molecule comprising an RNA region capable of forming an artificial stem-loop structure, wherein one of the annealing RNA sequences of the stem-loop structure comprises a nucleotide sequence essentially similar to at least part of the nucleotide sequence of a Δ12 desaturase encoding open reading frame, and wherein the second of said annealing RNA sequences comprises a sequence essentially similar to at least part of the complement of at least part of the nucleotide sequence of said Δ12 desaturase encoding open reading frame; and optionally;
   c) a DNA region involved in transcription termination and polyadenylation.
31. The method of item 30, wherein said DNA region comprises the nucleotide sequence of SEQ ID No 6.
32. The method of item 30, wherein said modifying of the fatty acid profile, comprises increasing the oleic acid content.
33. The method of item 30, wherein said plant expressible promoter is a seed-specific promoter.
34. The method of item 30, wherein said plant is oilseed rape.
35. A plant producing oil with modified fatty acid profile, said plant comprising a chimeric DNA, said chimeric DNA comprising the following operably linked parts:
   a). a plant-expressible promoter
   b). a DNA region, which when transcribed yields an RNA molecule comprising an RNA region capable of forming an artificial stem-loop structure, wherein one of the annealing RNA sequences of the stem-loop structure comprises a nucleotide sequence essentially similar to at least part of the nucleotide sequence of a Δ12 desaturase encoding open reading frame, and wherein the second of said annealing RNA sequences comprises a sequence essentially similar to at least part of the complement of at least part of the nucleotide sequence of said Δ12 desaturase encoding open reading frame; and optionally;
   c) a DNA region involved in transcription termination and polyadenylation.
36. The plant of item 35, wherein said DNA region comprises the nucleotide sequence of SEQ ID No 6.
37. The plant of item 35, wherein said plant expressible promoter is a seed-specific promoter.
38. The plant of item 35, wherein said plant is oilseed rape.

The following examples are presented in order to more fully illustrate the preferred embodiments of the invention. They should in no way be construed, however, as limiting the broad scope of the invention.

### EXAMPLE 1

### Experimental procedures

### Gene construction

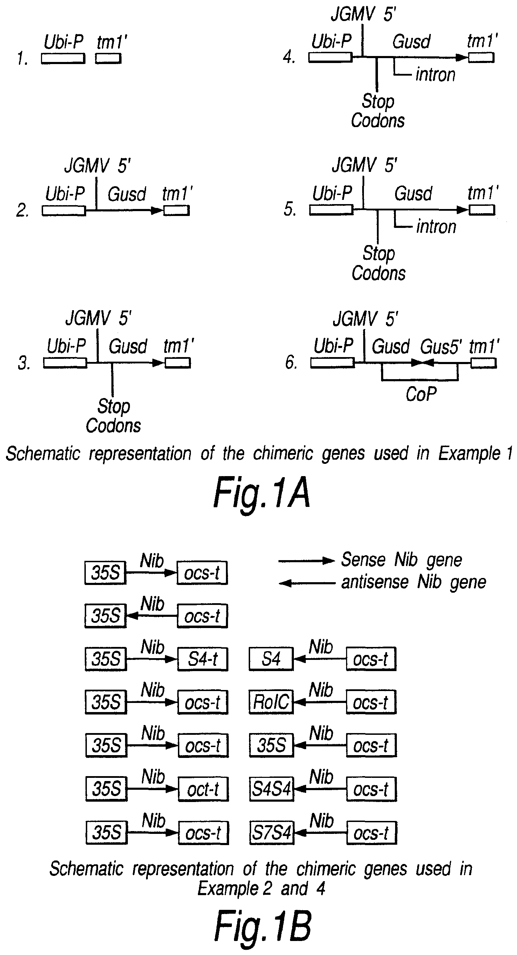

Standard gene cloning methods (Sambrook *et al.* 1989) were used to make the chimeric genes. A schematic representation of the constructs used is shown in Figures 1A and 1B.

The components for these constructs were:
* Cauliflower mosaic virus 35S promoter from the Cabb-JI isolate(35S) (Harpster *et al.,* 1988 )
* Octopine synthase terminator (ocs-t) (MacDonald *et al.,* 1991)
* Subterranean clover virus promoter No 4 (S4) (WO 9606932)
* Subterranean clover virus terminator No 4 (s4t) (WO 9606932)
* Subterranean clover virus double enhancer promoter No 4 (S4S4) (SEQ ID N° 5)
* Subterranean clover virus promoter No 4 with S7 enhancer (S7S4) (SEQ ID N° 4)
* Maize ubiquitin promoter (Ubi) (Christensen and Quail, 1996)
* Agrobacterium tumour morphology 1 gene terminator (tm1') (Zheng *et al. ,* 1991)
* the Nia gene of an Australian strain of Potato virus Y (Nia) (SEQ ID N° 1)
* a dysfunctional β-glucuronidase open reading frame encoding DNA (Gusd)(SEQ ID N° 2 from nucleotide 1 to nucleotide 1581)
* a modified 5' untranslated region (5'UTR) from Johnsongrass mosaic virus (JGMV5') (SEQ ID N° 3).
   This contains insertion of a *Ncol* site at the ATG start codon followed by three stopcodons in frame, and a *Pst*I site (for insertion of the intron as in constructs 4 and 5 of Figure 1A). In vector constructs 2 and 6 of Figure 1A, the Gusd open reading frame is inserted in at the *Nco*I site, removing the stop codons; in all other constructs of Fig 1A it is inserted downstream of the *Pst*I site.
* a castor bean catalase intron (Ohta et *al.,* 1990) as modified by Wang *et al.*(1997) ("intron").

The chimeric genes were constructed by operably assembling the different parts as schematically indicated in Figure 1A or Figure 1B and inserting the obtained chimeric genes in the T-DNA vectors pART27 and pART7 vectors (Gleave, 1992) between the left T-DNA border and the chimeric plant expressible neo gene.

The DNA encoding a dysfunctional β-glucuronidase open reading frame (GUSd) was obtained by deleting from a *gus* coding region the sequence between the two EcoRV restriction sites. For the construction of the chimeric gene encoding the RNA molecule comprising both sense and antisense nucleotide sequence to a β-glucuronidase gene, a sequence was added to the Gusd gene to be allow base pairing the 5'end over 558 bases resulting in a sequence as represented in SEQ ID N° 2. This sequence was cloned between the maize ubiquitin promoter and the tm1' terminator and inserted in a T-DNA vector.

T-DNA vectors were constructed which comprised a first and a second chimeric virus resistance gene, wherein the first chimeric gene consisted of:
1. a CaMV 35S promoter sequence, coupled to
2. in sense orientation, the nucleotide sequence from PVY encoding either
   - Vpg protein (see e.g. Genbank Accession Nr Z29526 from nucleotide 1013 to nucleotide 1583), or
   - part of the Cl protein (see e.g. Genbank Accession Nr M95491 from nucleotide 3688 to nucleotide 4215) or
   - Protease (Pro) (see e.g. EMBL Accession Nr D00441 from nucleotide 5714 to nucleotide 7009), followed by
3. the S4 terminator from subterranean clover mosaic virus, as described above.
The second chimeric gene consists of
1. a S4 promoter as described above, coupled to
2. in anti-sense orientation, the nucleotide sequence from PVY encoding either
   - Vpg protein , or
   - Cl protein or
   - Protease , followed by
3. the octopine synthase terminator as described above.
The sense and antisense sequences within one T-DNA vector were derived from the same PVY coding region.

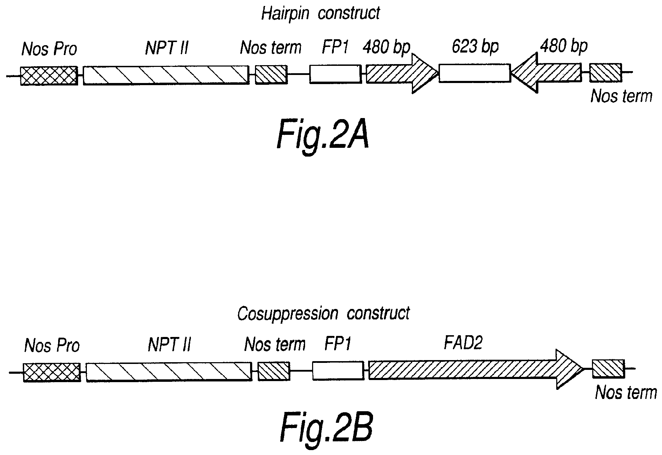

Also, T-DNA vectors were constructed for use in altering the fatty acid composition in oil (see Fig 2), comprising
1. a FP1 promoter (truncated seed specific napin promoter, containing sequences between -309 and +1, as described in Stalberg et al; linked to
2. the nucleotide sequence of SEQ ID No 6, comprising the 480 bp located 5' in the ORF encoding the Δ12 desaturase from Arabidopsis thaliana (Fad2) in sense orientation and in antisense orientation, linked by a 623 bp spacer sequence; followed by
3. the terminator from the nopaline synthase gene.

In addition, T-DNA vectors were constructed to evaluate the influence of a presence of an intron sequence in the chimeric genes encoding CoP constructs. To this end, constructs were made comprising:
1. a CamV35S promoter, followed by
2. the protease encoding ORF from PVY (see above) in sense orientation;
3. the sequence of SEQ ID No 7 (encoding the Flaveria trinervia pyruvate orthophospate dikinase intron 2)
4. the protease encoding ORF from PVY in antisense orientation; and
5. the octopine synthase gene terminator.

### Plant transformation

*Nicotiana tabacum* (W38) tissue was transformed and regenerated into whole plants essentially as described by Landsman *et al.* (1988). Rice (*Oryza sativa)* was transformed essentially as described by Wang *et al.* (1997).

### Rice supertransformation

Mature embryos from a rice plant expressing GUS and hygromycin phosphotransferase (HPT) activity were excised from mature seed and placed on callus inducing media for 7 weeks. Calli were recovered from these cultures, incubated with Agrobacteria containing various binary vector constructs for 2 days, then placed on callusing media containing hygromycin, bialaphos and Timentin™. During the next four weeks hygromycin and bialaphos resistant calli developed. These callus lines were maintained on hygromycin and bialaphos containing media for a further 2 months before being assayed for GUS activity.

### GUS Assay

Rice calli were tested for GUS activity using the histochemical stain X-glucuronide or the fluorogenic substrate 4-methyl-umbeliferone glucuronide (MUG) essentially as described by Jefferson *et al.* (1987).

### Example 1. Comparison of chimeric genes comprising only antisense, only sense, or both sense and antisense (Complimentary Pair (CoP)) sequence for reduction in phenotypic expression of an integrated β-glucuronidase gene.

Transgenic rice tissue expressing β-glucuronidase (GUS) from a single transgene (and hygromycin resistance from a *hph* gene) (lines V10-28 and V10-67) was supertransformed using vectors that contained the *bar* gene conferring phosphinothricin resistance and various sense, antisense and CoP constructs (see Figure 1A) derived from a crippled GUS (GUSd) gene. The supertransformed tissue was maintained on hygromycin and bialaphos selection media for 3 weeks then analyzed for GUS activity. A crippled GUS gene was used so that expression from this gene would not be superimposed on the endogenous GUS activity.

The figures in Table 2 represent the rate of MU production measured by absorption at 455 nm, with excitation at 365 nm of 1.5 µg ot total protein in a reaction volume of 200 µl. The rate was measured over 30 min at 37°C. The reading for non-transgenic rice calli was 0.162. The figures in bracket which follow the description of the introduced construct refer to Figure 1A.

The results (Table 2) showed that supertransformation with the binary vector containing the *bar* gene without the GUSd gene had no silencing effect on the endogenous GUS activity. Supertransformation with GUSd in a sense or antisense orientation, with or without an intron or an early stop codon, showed some degree of reduction (in about ∼25% of the analysed calli) of the endogenous GUS activity (see last two rows in Table 2 representing the percentage of analysed calli with a MUG assay reading of less than 2.000). However, supertransformation with a CoP construct gave in about 75% to 100% of the analysed calli, reduction of the endogenous GUS activity. This CoP construct was designed so that the 3' end of the mRNA produced could form a duplex with the 5' end of the transcript to give a "pan-handle" structure.

These data show that a complimentary pair can be made using one self-annealing transcript, that this design is much more effective than a conventional sense or antisense construct, and that the approach can be used to reduce the phenotypic expression of genes present in a plant cell.

**Table 2. MUG assay of Super transformed Rice Calli**

| | Vector cassette | Sense | Sense + Stop | Sense + stop+ | Antisense + stop + | Inverted repeat |
|---|---|---|---|---|---|---|
| | (1) | (2) | (3) | intron (4) | intron (5) | CoP (6) |
| V10-28 | 121,0 | 97,45 | 38,43 | 38,88 | 0,290 | 0,565 |
| | 45,58 | 6,637 | 64,16 | 115,5 | 0,572 | 0,316 |
| | 99,28 | 71,60 | 149,2 | 133,0 | 37,2 | 0,351 |
| | 26,17 | 0,224 | 0,955 | 98,46 | 53,94 | 0,210 |
| | 92,21 | 0,321 | 68,32 | 0,502 | 105,5 | 0,701 |
| | 108,8 | 5,290 | 105,6 | 39,35 | 56,73 | 0,733 |
| | 6,432 | 0,9460 | 136,6 | 1,545 | 60,36 | 2,103 |
| | 90,80 | 32,44 | 140,4 | 10,36 | 71,12 | 119,8 |
| | 98,24 | 128,8 | 62,38 | 111,6 | 13,17 | 0,717 |
| | 93,76 | 31,28 | 17,79 | 14,42 | 0,424 | 0,398 |
| | | 5,023 | | 88,06 | 26,98 | 0,315 |
| | | 40,27 | | 52,28 | 115,5 | 0,270 |
| | | 36,40 | | 30,26 | 149,7 | 16,78 |
| | | 53,24 | | 107,5 | 66,75 | 67,28 |
| | | 29,97 | | 26,75 | 145,8 | 0,217 |
| | | 89,06 | | 105,1 | 0,534 | 0,208 |
| | | 0,256 | | 135,1 | 9,4 | |
| | | 68,23 | | 95,04 | 35,33 | |
| | | 5,481 | | 71,5 | | |
| V10-67 | 318,8 | 93,43 | 0,199 | 31,82 | 1,395 | 0,472 |
| | 109,5 | 73,19 | 0,197 | 58,08 | 152,4 | 0,256 |
| | 30,35 | 128,1 | 0,157 | 56,32 | 67,42 | 0,296 |
| | 40,04 | 1,506 | 128 | 44,62 | 12,11 | 0,452 |
| | 228 | 140,6 | 130,3 | 0,454 | 0,668 | 0,422 |
| | 23,05 | 1,275 | 196,2 | 17,32 | 23,34 | 0,196 |
| | 241,2 | 0,272 | 12,43 | 73,2 | 76,10 | 0,294 |
| | 118,5 | 0,209 | 140,0 | 20,32 | 130,1 | 0,172 |
| | 11,27 | 42,05 | 90,13 | 107,4 | 0,841 | 0,436 |
| | 110,6 | 117,5 | 157,4 | 0,453 | 66,12 | 0,398 |
| | 19,29 | 118,9 | 0,518 | 87,81 | 136,9 | 0,242 |
| | 121,0 | 21,44 | 0,231 | 0,299 | 67,92 | |
| | 115,1 | 155,0 | 116,1 | 0,206 | 50,32 | |
| | 77,1 | 190,9 | 43,18 | 12,47 | 170,3 | |
| | 106,1 | 0,773 | 31,06 | 0,213 | 108,9 | |
| | 73,12 | 0,146 | | 11,15 | 1,241 | |
| | 29,97 | | | 19,22 | 4,092 | |
| | 50,11 | | | | 169,6 | |
| | 80,34 | | | | 76,88 | |
| | 117,8 | | | | 22,08 | |
| | 159,1 | | | | 91,6 | |
| | 67,52 | | | | 7,855 | |
| | 92,32 | | | | 69,76 | |
| | 27,97 | | | | 0,822 | |
| V10-28 | 0% | 21% | 10% | 10,5% | 22% | 75% |
| V10-67 | 0% | 37,5% | 33% | 29,5% | 21% | 100% |

### Example 2. Comparison of the efficiency of using chimeric genes comprising only antisense genes, only sense genes, or both genes simultaneously for obtaining virus resistance in transgenic plants.

Gene constructs were made using the PVY protease encoding sequence ( SEQ ID N° 1) in a sense orientation, an antisense orientation and in a complimentary pair (CoP) orientation, where the T-DNA comprised both the sense and antisense chimeric genes each under control of their own promoter. In all three arrangements the CaMV35S promoter was used. Five different versions of CoP constructs were made in which the second promoter was either the CaMV35S promoter, the S4 promoter, the double S4 promoter, the S7 enhanced S4 promoter, or the vascular specific *rol*C promoter (see Figure 1 B).

These constructs were transformed into tobacco (via *Agrobacterium* mediated DNA transfer) and approximately 25 independently transformed plants were recovered per chimeric gene construct. The transgenic plants were transferred to soil and maintained in the greenhouse. About 1 month after transplanting to soil, the plants were inoculated manually with potato virus Y, using standard application methods. Two and four weeks later the plants were scored for virus symptoms. The results (Table 3) showed that after 1 month, 2 on 27 plants comprising only the sense gene, and 1 on 25 plants comprising only the antisense gene showed no symptoms.

In contrast respectively 11 on 24 (35S-Nia/S4-antisenseNia construct), 7 on 25 (35S-Nia/RolC-antisenseNia construct), 10 on 27 (35S-Nia/35S-antisenseNia), 7 on 26 (35S-Nia/S4S4-antisenseNia construct), and 7 on 25 (35S-Nia/S7S4-antisenseNia construct) plants which contained both the sense and antisense genes, showed no symptoms. Plants that showed no symptoms were considered to be showing extreme resistance to PVY. They continued to show no symptoms for a further 2 months of monitoring (indicated as Extreme Resistant (ER) in Table 3). Some other plants, particularly those containing CoP constructs, showed a delay and restriction of symptoms. They showed no symptoms 2 weeks after inoculation but showed some minor lesions in some plants after 4 weeks. These plants were clearly much less effected by PVY than non-transgenic or susceptible tobaccos and were scored as resistant (indicated as ER* in Table 3).

**Table 3 Resistance to PVY infection of transgenic tobacco plants comprising either the sense chimeric PVY protease construct, the antisense chimeric PVY protease construct, or both (different CoP constructs).**

| Sense gene | Antisense gene | Extreme Resistant plants (ER) | "Resistant" plants (ER*) | Total number of transgenic plants |
|---|---|---|---|---|
| 35S-Nia | | 2 | 2 | 27 |
| | 35S-AntisenseNia | 1 | 0 | 25 |
| 35S-Nia | 35S-AntisenseNia | 10 | 2 | 27 |
| 35S-Nia | S4-AntisenseNia | 11 | 2 | 24 |
| 35S-Nia | RolC-AntisenseNia | 7 | 3 | 25 |
| 35S-Nia | S4S4-AntisenseNia | 7 | 7 | 26 |
| 35S-Nia | S7S4-AntisenseNia | 7 | 4 | 25 |

The data show that using CoP constructs results in a much higher frequency of transgenic plants with extreme resistance and resistance than by using either sense or antisense constructs alone.

Next, the copy number of the transgenes in the virus resistant transgenic plants was determined. Therefore, DNA was extracted from all the transgenic plants showing extreme resistance or resistance. DNA was also extracted from five susceptible plants for each construct. The DNA was examined for gene copy number using Southern analysis. The data (Table 4) showed that the genomes of some of the CoP plants showing extreme resistance, particularly the 35S-Nia/S4-AntisenseNia plants, only contained a single copy of the gene construct.

**Table 4. Copy number of transgenes comprising sense chimeric PVY protease construct, the antisense chimeric PVY protease construct, or both (different CoP constructs) in extreme resistant, resistant and susceptible plants.**

| Sense gene | Antisense gene | Extreme Resistant plants (ER) | "Resistant" plants (ER*) | Susceptible plants |
|---|---|---|---|---|
| 35S-Nia | | 6 | 1 | 1/1/1/1/1 |
| | 35S-AntisenseNia | 4 | - | 1/8/0/2/1 |
| 35S-Nia | 35S-AntisenseNia | 3/1/2/3/6/3/2/4/2/3 | 1/1 | 1/2/2/6/1 |
| 35S-Nia | S4-AntisenseNia | 2/4/1/3/2/4/6/1/1/3/1 | 2/6 | 5/8/2/3 |
| 35S-Nia | RolC-AntisenseNia | 6/7/6/7/7/7/6 | 2/1 | 2/2/2/1/2 |
| 35S-Nia | S4S4-AntisenseNia | 1/2/4/5/2/2/2 | 1/1/2/1/1/1/1 | 1/1/7/1/1 |
| 35S-Nia | S7S4-AntisenseNia | 2/4/12/5/2/2/7 | 3/2/1/2 | 1/1/3/1/1 |

### Example 3 Inheritance of extreme resistance in plants from Example 2.

Plants from example 2 were allowed to self fertilise and their seeds were collected. Seeds originating from plants showing extreme resistance and low transgene copy number for CoP constructs 35S-Nia/S4-AntisenseNia and 35S-Nia/35S-AntisenseNia, and seeds from the sense and the antisense plants showing extreme resistance, were germinated and grown in the glasshouse. Plants were also grown from seed collected from two susceptible CoP lines, two susceptible sense gene only lines and two susceptible antisense gene only lines. Twenty plants from each line were selected for overall uniformity of size and development stage, put into individual pots, allowed to recover for one week, then inoculated with PVY. The plants were scored for virus symptoms 2,4, and 7 weeks after inoculation. The results (Table 5) showed that all eight plant lines of 35S-Nia/S4-antisenseNia and 35S-Nia/35S-antisenseNia containing one or two gene copies showed an about 3:1 segregation ratio of extreme resistance : susceptible. The progeny of the single antisense gene only line that had given extreme resistance at T₀, and the progeny of the extremely resistant sense plant containing one gene copy, gave abnormal segregation ratios (2: 18; ER:susceptible). The progeny of the one sense plant that gave extreme resistance and contained 6 gene copies gave a ∼ 3:1 ratio (ER: susceptible). All the progeny of the susceptible T₀ plants showed complete susceptibility to PVY.

These data shows extreme resistance from CoP constructs gives stable expression of the resistance which is inherited in a Mendelian way. This also indicates that, in these lines the PVY CoP gene loci are ∼100% effective at conferring extreme resistance whereas the transgene loci in the antisense line and one of the two sense lines are only partially effective at conferring extreme resistance.

**Table 5.**

| | 35S Sense Nia and S4 Antisense Nia | | 35S Sense Nia and S4 Antisense Nia | | 35S Sense Nia | | 35SAntisenseNia | |
|---|---|---|---|---|---|---|---|---|
| | Copy N° | T1 ER:S | Copy N° | T1 ER:S | Copy N° | T1 ER:S | Copy N° | T1 ER:S |
| ER plant 1 | 1 | 15:5 | 1 | 16:4 | 6 | 17:3 | 4 | 2:18 |
| 2 | 1 | 12:8 | 2 | 15:5 | 1 | 2:18 | | |
| 3 | 1 | 14:6 | 2 | 16:4 | | | | |
| 4 | 1 | 15:5 | 2 | 16:4 | | | | |
| Susceptible | | | | | | | | |
| Plant 1 | 8 | 0:20 | 1 | 0:20 | 1 | 0:20 | 1 | 0:20 |
| 2 | 2 | 0:20 | 1 | 0:20 | 1 | 0:20 | 8 | 0:20 |

### Example 4: Extreme virus resistant transgenic tobacco with different components (sense gene and antisense gene) in different loci within the transgenic plant.

PVY susceptible plants containing the sense transgene (which contained single transgene copies; see Table 6) were crossed with PVY susceptible plants containing the antisense transgene (which had also been analysed for copy number by Southern analysis; see Table 6). Twenty of the resulting progeny per cross were propagated in the glasshouse, then inoculated with PVY and scored for virus infection as described in Example 2. The progeny from the crosses (between single genes/loci containing plants) would be expected to be in the following ratio: ¼ sense gene alone, ¼ antisense gene alone, ¼ comprising both sense and antisense genes, and ¼ comprising no genes at all. The results (Table 4) show that, with one exception, a proportion of the progeny from all the successful crosses showed extreme resistance, whereas none of the progeny from selfed sense or selfed antisense plants showed extreme resistance. The one cross that gave no extremely resistant progeny was derived from the parent plant Antisense 2 (As2) which contained 8 copies of the antisense gene. All twenty progeny plants from crosses Sense 1 (S1)(male) x Antisense 1 (As1) (female) and Sense 3 (S3) (female) x Antisense 4 (As4) (male) were examined by Southern analysis. The results showed that in both crosses, the plants that showed extreme resistance (or in one case resistance) contained both the sense and antisense genes, whereas plants with no transgenes (nulls), or sense or antisense genes alone, were all susceptible to PVY. To further confirm this absolute correlation between the presence of a complimentary pair (sense with antisense genes) within a plant and extreme resistance, all progeny plants showing extreme resistance were analysed by Southern blots. The results showed that every extremely resistant or resistant plant contained both sense and antisense genes.

These data show that a complimentary pair gives resistance or extreme resistance even when the genes encoding the sense and antisense genes are not co-located in the genome. The "complimentary pair phenomenon" is not simply due to increased transgene dosage as it would be expected that ¼ of the selfed progeny would be homozygous and thus have double the gene dosage, yet they were susceptible.

**Table 6: PVY resistance of the progeny plants resulting from crosses between susceptible transgenic tobacco plants comprising the 35S-senseNia gene (S-lines) and susceptible transgenic tobacco plants comprising the 35S-antisenseNia gene (As-lines).**

| Male parent | Female parent | Extreme Resistant plants (ER) | "Resistant" plants (ER*) |
|---|---|---|---|
| S1 | As1 | 8 | |
| S1 | As4 | 6 | 5 |
| S2 | As4 | 1 | 3 |
| S3 | As4 | 3 | 3 |
| S4 | As1 | 7 | 1 |
| S3 | As5 | 1 | 2 |
| S4 | As2 | 0 | |
| S4 | As4 | 2 | 0 |
| S5 | As4 | 9 | 4 |
| S5 | As5 | 2 | 3 |
| As4 | As4 | 0 | |
| As5 | As5 | 0 | |
| S2 | S2 | 0 | |
| S4 | S4 | 0 | |

Extreme resistant plants showed no symptoms of PVY infection after 7 weeks. Resistant plants showed very minor lesions 7 weeks after PVY infection.
S1, S2, S3, S4 and S5 are PVY susceptible transgenic tobacco plants comprising the 35S-senseNia gene construct which all have one copy of the transgene integrated.

As1, As2, As4 and As5 are PVY susceptible transgenic tobacco plants comprising the 35S-antisenseNia gene construct which have respectively 1, 8, 2 and 1 copies of the transgene integrated.

### Example 5. Evaluation of the use of different viral genes as target nucleic acid sequences in obtaining extreme virus resistant genes.

The T-DNA vectors comprising first and second chimeric virus resistance genes based on sequences derived from the coding region for protease, Vpg or Cl proteins from PVY as described in this application, were used to obtain transformed tobacco plants, which were subsequently challenged with PVY. The results are summarized in the following table:

**Table 7.**

| Construct | Number of immune plants/ Number of independent transgenic plants | |
|---|---|---|
| | Replica 1 | Replica 2 |
| 35S-Pro sense /S4-Pro antisense | 11/24 | 7/25 |
| 35S-Vpg sense /S4-Vpg antisense | 8/20 | 6/18 |
| 35S-Cl sense /S4-Cl antisense | 2/23 | 1/20 |

### Example 6. Intron enhanced silencing

The T-DNA vectors comprising the chimeric genes encoding the CoP constructs wherein an intron (Flaveria trinervia pyruvate orthophosphate dikinase intron 2) has been inserted in either the sense orientation or the antisense orientation, between the sense and antisense sequences corresponding to the protease encoding ORF from PVY (as described elsewhere in this application) were used to obtain transformed tobacco plants, which were subsequently challenged with PVY. The results are summarized in the following table:

**Table 8.**

| Construct | Number of immune plants/ Number of independent transgenic plants |
|---|---|
| 35S-Pro(sense)-intron(sense)-Pro(antisense)-Ocs-t | 22/24 |
| 35S-Pro(sense)-intron(antisense)-Pro(antisense)-Ocs-t | 21/24 |

### Example 7. Modifying oil profile using CoP constructs in Arabidopsis.

T-DNA vectors for modifying the fatty acid composition in oil, extracted from crushed seeds as described elsewhere in this application were used to introduce the chimeric gene encoding the CoP construct for reducing the expression (see Fig 2A; SEQ ID No 6) the Δ12 desaturase gene (Fad2) in Arabidopsis thaliana.

For comparison of the efficiency, transgenic Arabidopsis plants were generated wherein the Fad2 gene expression was reduced by a plain cosuppression construct, comprising the FPI seed-specific promoter coupled to the complete ORF from the Δ12 desaturase gene (Fad2) in Arabidopsis thaliana and the nopaline synthase promoter (see Fig 2B).

As control plants, transgenic Arabidopsis transformed by unrelated T-DNA constructs were used.

Seeds were harvested, crushed and extracted and the percentage of the major fatty acids in the oil was determined by methods available in the art. The results, which are the mean of two readings, are summarized in Table 9.

**Table 9.**

| **Sample** | **Peak Names -------->** | | | | | | | | | | | | |
|---|---|---|---|---|---|---|---|---|---|---|---|---|---|
| **Name** | **Myristic** | **Palmitic** | **Palmitoleic** | **Stearic** | **Oleic** | **Linoleic** | **Linolenic** | **20:0** | **20:1** | **22:0** | **22:1** | **24:0** | **C18:1/(C18:2 + C18:3)** |
| Hairpin 1.1 | 0.00 | 6.06 | 0.52 | 3.21 | 56.65 | 7.50 | 6.82 | 1.46 | 16.02 | 0.00 | 1.76 | 0.00 | 3.95 |
| Hairpin 1.2 | 0.12 | 6.86 | 0.39 | 3.40 | 51.28 | 10.00 | 8.73 | 1.64 | 15.60 | 0.00 | 1.97 | 0.00 | 2.74 |
| Hairpin 1.3 | 0.11 | 8.47 | 0.50 | 3.49 | 21.64 | 28.99 | 18.51 | 2.02 | 14.19 | 0.00 | 2.09 | 0.00 | 0.46 |
| Hairpin 1.4 | 0.00 | 6.14 | 0.50 | 3.37 | 51.70 | 9.77 | 8.02 | 1.73 | 16.04 | 0.00 | 2.05 | 0.67 | 2.91 |
| Hairpin 2.1 | 0.06 | 5.19 | 0.43 | 3.33 | 54.84 | 5.52 | 7.76 | 1.77 | 18.50 | 0.34 | 1.83 | 0.45 | 4.13 |
| Hairpin 2.2 | 0.04 | 7.67 | 0.46 | 3.75 | 19.60 | 28.29 | 18.64 | 2.55 | 15.96 | 0.19 | 2.28 | 0.56 | 0.42 |
| Hairpin 3.1 | 0.00 | 7.99 | 0.53 | 3.62 | 19.52 | 28.41 | 19.24 | 2.32 | 15.14 | 0.00 | 2.23 | 0.99 | 0.41 |
| Hairpin 3.2 | 0.09 | 7.00 | 0.54 | 3.69 | 49.02 | 11.03 | 9.64 | 1.71 | 14.94 | 0.00 | 1.72 | 0.62 | 2.37 |
| Hairpin 3.3 | 0.00 | 5.68 | 0.49 | 3.98 | 46.19 | 12.82 | 9.71 | 2.10 | 16.70 | 0.00 | 1.94 | 0.39 | 2.05 |
| Hairpin 3.4 | 0.17 | 7.19 | 0.77 | 3.69 | 45.90 | 11.86 | 10.65 | 1.84 | 15.39 | 0.00 | 1.90 | 0.65 | 2.04 |
| Hairpin 3.5 | 0.00 | 6.45 | 0.48 | 3.26 | 51.76 | 8.13 | 10.04 | 1.51 | 16.08 | 0.00 | 1.92 | 0.36 | 2.85 |
| Hairpin 3.6 | 0.08 | 7.51 | 0.23 | 3.59 | 19.97 | 29.13 | 20.12 | 2.15 | 14.54 | 0.29 | 2.02 | 0.36 | 0.41 |
| Hairpin 3.7 | 0.14 | 7.20 | 0.78 | 2.90 | 26.37 | 24.81 | 17.18 | 1.92 | 15.50 | 0.36 | 2.30 | 0.53 | 0.63 |
| Hairpin 3.8 | 0.11 | 6.34 | 0.46 | 3.23 | 38.58 | 15.25 | 13.54 | 1.89 | 16.91 | 0.00 | 2.36 | 1.34 | 1.34 |
| Hairpin 3.9 | 0.00 | 6.47 | 0.49 | 3.32 | 47.59 | 11.44 | 9.63 | 1.68 | 15.96 | 0.00 | 1.88 | 1.55 | 2.26 |
| Hairpin 3.10 | 0.00 | 6.77 | 0.56 | 3.48 | 53.30 | 7.57 | 9.34 | 1.55 | 15.65 | 0.00 | 1.79 | 0.00 | 3.15 |
| Hairpin 3.11 | 0.00 | 7.05 | 0.59 | 3.61 | 53.62 | 8.87 | 8.36 | 1.55 | 14.35 | 0.00 | 1.99 | 0.00 | 3.11 |
| Hairpin 3.12 | 0.05 | 8.32 | 0.36 | 3.85 | 18.48 | 29.24 | 19.94 | 2.48 | 14.75 | 0.00 | 2.28 | 0.26 | 0.38 |
| Hairpin 4.1 | 0.09 | 6.97 | 0.59 | 3.61 | 53.64 | 8.40 | 8.44 | 1.60 | 15.00 | 0.00 | 1.66 | 0.00 | 3.19 |
| Hairpin 4.2 | 0.07 | 6.81 | 0.22 | 3.27 | 55.06 | 9.16 | 8.71 | 1.26 | 13.63 | 0.19 | 1.33 | 0.30 | 3.08 |
| Hairpin 4.3 | 0.04 | 6.81 | 0.50 | 3.47 | 46.21 | 10.67 | 11.52 | 1.81 | 16.50 | 0.00 | 1.88 | 0.58 | 2.08 |
| Hairpin 5.1 | 0.00 | 8.30 | 0.23 | 3.71 | 17.72 | 28.92 | 20.63 | 2.38 | 14.77 | 0.00 | 2.41 | 0.92 | 0.36 |
| Hairpin 5.2 | 0.19 | 7.15 | 1.55 | 3.56 | 44.58 | 11.44 | 11.59 | 1.77 | 15.67 | 0.00 | 1.84 | 0.65 | 1.94 |
| Hairpin 5.3 | 0.10 | 6.49 | 0.40 | 3.72 | 54.19 | 7.01 | 7.89 | 1.74 | 15.91 | 0.00 | 1.92 | 0.62 | 3.64 |
| Hairpin 5.5 | 0.12 | 6.58 | 0.51 | 3.84 | 54.48 | 6.16 | 7.23 | 1.77 | 16.50 | 0.42 | 1.90 | 0.48 | 4.07 |
| Hairpin 5.6 | 0.00 | 6.67 | 0.50 | 3.66 | 46.32 | 11.56 | 10.48 | 1.83 | 15.99 | 0.00 | 2.15 | 0.84 | 2.10 |
| Hairpin 5.7 | 0.00 | 5.50 | 0.51 | 3.58 | 57.33 | 4.75 | 5.91 | 1.75 | 18.03 | 0.00 | 1.88 | 0.76 | 5.38 |
| Hairpin 5.8 | 0.16 | 6.55 | 1.53 | 3.54 | 48.52 | 9.91 | 8.97 | 1.78 | 16.39 | 0.00 | 1.84 | 0.81 | 2.57 |
| Hairpin 6.1 | 0.10 | 6.35 | 0.57 | 3.48 | 59.00 | 4.77 | 6.26 | 1.48 | 15.95 | 0.00 | 1.80 | 0.25 | 5.35 |
| Hairpin 6.2 | 0.10 | 7.98 | 0.37 | 4.06 | 20.96 | 29.01 | 18.69 | 2.38 | 13.63 | 0.20 | 2.03 | 0.60 | 0.44 |
| Hairpin 6.5 | 0.08 | 6.21 | 0.63 | 3.61 | 60.05 | 5.07 | 5.27 | 1.55 | 15.20 | 0.00 | 1.69 | 0.66 | 5.81 |
| Columbia pBin 19 control | 0.08 | 8.81 | 0.47 | 3.51 | 17.07 | 30.31 | 20.94 | 1.78 | 14.56 | 0.00 | 2.17 | 0.28 | 0.33 |
| Cosuppresion 1.1 | 0.08 | 8.16 | 0.62 | 3.71 | 26.16 | 23.77 | 18.15 | 2.06 | 14.65 | 0.17 | 1.89 | 0.57 | 0.62 |
| Cosuppresion 1.2 | 0.00 | 8.49 | 0.53 | 3.65 | 17.90 | 29.93 | 20.36 | 2.34 | 14.25 | 0.00 | 2.33 | 0.23 | 0.36 |
| Cosuppresion 1.3 | 0.07 | 6.65 | 0.40 | 3.42 | 38.34 | 15.25 | 14.16 | 1.91 | 17.19 | 0.31 | 1.94 | 0.35 | 1.30 |
| Cosuppresion 1.4 | 0.00 | 8.22 | 0.57 | 3.82 | 18.27 | 28.82 | 19.63 | 2.56 | 14.83 | 0.00 | 2.46 | 0.83 | 0.38 |
| Cosuppresion 1.5 | 0.00 | 7.51 | 0.52 | 3.84 | 34.59 | 17.90 | 14.64 | 2.18 | 16.27 | 0.00 | 2.02 | 0.54 | 1.06 |
| Cosuppresion 1.6 | 0.07 | 7.44 | 0.47 | 3.16 | 23.97 | 27.32 | 17.29 | 2.03 | 15.52 | 0.18 | 2.22 | 0.33 | 0.54 |
| Cosuppresion 2.1 | 0.07 | 7.46 | 0.43 | 3.00 | 23.91 | 27.21 | 17.79 | 1.84 | 15.27 | 0.30 | 2.14 | 0.58 | 0.53 |
| Cosuppresion 2.2 | 0.00 | 8.19 | 0.55 | 4.22 | 18.59 | 28.31 | 18.80 | 2.77 | 15.51 | 0.00 | 2.46 | 0.58 | 0.39 |
| Cosuppresion 2.3 | 0.00 | 8.71 | 0.47 | 3.48 | 19.21 | 30.06 | 19.49 | 2.03 | 13.78 | 0.00 | 2.15 | 0.63 | 0.39 |
| Cosuppresion 3.1 | 0.06 | 7.57 | 0.50 | 3.83 | 32.24 | 20.00 | 15.66 | 2.06 | 15.65 | 0.34 | 1.85 | 0.23 | 0.90 |
| Cosuppresion 4.1 | 0.00 | 7.29 | 0.43 | 3.55 | 30.26 | 21.17 | 17.06 | 2.01 | 16.08 | 0.00 | 1.92 | 0.25 | 0.79 |
| Cosuppresion 4.2 | 0.08 | 8.02 | 0.53 | 3.62 | 33.04 | 20.04 | 15.68 | 1.80 | 14.72 | 0.00 | 1.88 | 0.58 | 0.92 |
| Cosuppresion 4.3 | 0.07 | 8.35 | 0.54 | 3.85 | 30.02 | 21.72 | 16.78 | 2.01 | 14.25 | 0.00 | 1.92 | 0.49 | 0.78 |
| Cosuppresion 4.4 | 0.06 | 6.98 | 0.53 | 3.62 | 43.38 | 13.24 | 12.77 | 1.74 | 15.37 | 0.30 | 1.67 | 0.33 | 1.67 |
| Cosuppresion 4.5 | 0.13 | 7.84 | 0.52 | 3.76 | 33.76 | 18.16 | 16.21 | 1.89 | 14.96 | 0.35 | 1.85 | 0.57 | 0.98 |
| Cosuppresion 4.6 | 0.11 | 8.18 | 0.32 | 3.58 | 19.72 | 29.19 | 20.26 | 2.04 | 13.92 | 0.29 | 1.84 | 0.55 | 0.40 |
| Cosuppresion 4.7 | 0.11 | 7.88 | 0.39 | 3.75 | 27.40 | 22.85 | 17.44 | 2.08 | 15.29 | 0.00 | 2.04 | 0.76 | 0.68 |
| Cosuppresion 4.8 | 0.13 | 7.56 | 0.41 | 3.46 | 32.27 | 20.50 | 15.45 | 1.90 | 15.47 | 0.00 | 2.02 | 0.83 | 0.90 |
| Cosuppresion 4.9 | 0.09 | 7.46 | 0.29 | 3.75 | 36.11 | 16.96 | 15.74 | 1.92 | 15.38 | 0.31 | 1.74 | 0.25 | 1.10 |
| Cosuppresion 5.1 | 0.10 | 7.68 | 0.34 | 3.88 | 36.00 | 16.77 | 15.38 | 1.90 | 15.44 | 0.32 | 1.82 | 0.36 | 1.12 |
| Cosuppresion 5.2 | 0.08 | 7.56 | 0.25 | 3.58 | 26.10 | 25.11 | 17.79 | 1.96 | 15.03 | 0.30 | 1.72 | 0.54 | 0.61 |
| Cosuppresion 5.3 | 0.08 | 7.38 | 0.20 | 3.56 | 42.24 | 13.33 | 13.32 | 1.76 | 15.19 | 0.16 | 1.61 | 1.18 | 1.59 |
| Cosuppresion 6.1 | 0.08 | 8.04 | 0.50 | 3.68 | 31.37 | 20.29 | 17.17 | 1.84 | 14.31 | 0.00 | 1.76 | 0.95 | 0.84 |
| Cosuppresion 6.2 | 0.00 | 8.50 | 0.51 | 3.91 | 18.59 | 29.33 | 19.66 | 2.46 | 14.75 | 0.00 | 2.28 | 0.00 | 0.38 |
| Control c24 pGNAP-p450 | 0.07 | 8.30 | 0.10 | 4.78 | 19.68 | 25.91 | 20.56 | 2.97 | 15.29 | 0.31 | 1.79 | 0.24 | 0.42 |

Analysis of the results indicates that transgenic plants harboring a CoP construct (indicated as "hairpin x.x" in the table) have a higher frequency of plants with oil wherein the increase in oleic acid and concomitant decrease in linolenic and linoleic acid is significant than in transgenic plants harbouring cosuppression constructs.. Moreover the absolute levels of increase, respectively decrease are higher respectively lower than in transgenic plants harbouring cosuppression constructs.

### Example 7. Modifying oil profile using CoP constructs in Brassica.

The T-DNA vector harbouring the chimeric gene encoding the CoP construct described in Example 6 is introduced in *Brassica* oilseed rape. Seeds harvested from the transgenic *Brassica sp.* are crashed and oil extracted and the composition of the fatty acids in the oil is analysed.

Oil from transgenic *Brassica sp.* harbouring the CoP construct have significantly increased oleic acid content and decreased linoleic and linolenic acid content. A T-DNA vector harbouring a chimeric gene encoding a CoP construct similar to the one described in Example 6, but wherein the sequence of the sense and antisense region corresponding to the Δ12 desaturase encoding ORF is based on a homologous ORF from *Brassica spp.* is constructed and introduced in Brassica oilseed rape.

The sequence of Brassica spp ORFs homologous to Δ12 desaturase encoding ORF from Arabidopsis are available from Genbank database under Accession nrs AF042841 and AF124360.

Seeds harvested from the transgenic *Brassica sp.* are crashed and oil extracted and the composition of the fatty acids in the oil is analysed. Oil from transgenic *Brassica sp.* harbouring the CoP construct have significantly increased oleic acid content and decreased linoleic and linolenic acid content.

### Example 8. Suppression of an endogenous rust resistance gene in flax.

A CoP construct for suppression of the endogenous rust resistance gene was made consisting of
1. a CaMV35S promoter; operably linked to
2. part of an endogenous rust resistance gene (n) from flax (about 1500 bp long) in the sense orientation; ligated to
3. a similar part of the endogenous rust resistance gene from flax (about 1450 bp long) in antisense orientation so that a perfect inverted repeat without spacer sequence is generated wherein each repeat is about 1450 bp long; followed by
4. a nos terminator.

Plain antisense constructs were made using a similar fragment as described sub 3 above inserted between a CaMV35S promoter and a nos terminator.

Flax plants containing the n gene (which gives resistance to a strain of flax rust) were transformed by these CoP and antisense constructs. If suppression occurs, the plants become susceptible to the rust strain. If the construct has no effect, the transformed plants remain resistant to the rust strain.

**Results**

| | |
|---|---|
| ngc-b sense/antisense | 3 suppressed out of 7 |
| ngc-b antisense | 0 suppressed out of 12 |

While the invention has been described and illustrated herein by references to various specific material, procedures and examples, it is understood that the invention is not restricted to the particular material, combinations of material, and procedures selected for that purpose. Numerous variations of such details can be implied and will be appreciated by those skilled in the art.

### REFERENCES

An et al. (1996) The Plant Cell 8, 15-30
Barry et al. (1993) Proc Natl Acad Sci 90, 4557-4561
Baulcombe (1996) Plant Cell 8, 1833-1844
Braun and Hemenway (1992) Plant Cell 4, 735-744
Brederode et al. (1995) Virology 207, 467-474
Carr et al. (1992) Mol. Plant-Microb. Interact. 5, 397-404
Christensen and Quail (1996) Transgenic Research 5, 213-218
Croy Plant Molecular Biology Labfax (1993) BIOS Scientific Publications Ltd (UK) and Blackwell Scientific Publications, UK.
de Carvalho Niebel et al. (1995) Plant Cell 7, 347-358
English et al. (1996) Plant Cell 8, 179-188
Fire et al. (1998) Nature 391, 806-811
Fromm et al. (1990) Bio/Technology 8: 833
Gleave, (1992)Plant Mol. Biol. 20: 1203-1207
Goodwin et al. (1996) Plant Cell 8, 95-105
Gordon-Kamm et al. (1990)The Plant Cell 2: 603
Harpster et al. (1988) Mol. Gen. Genet. 212, 182-190
Hobbs et al. (1990) Plant Mol. Biol. 15, 851-864
Hudspeth et al. (1989) Plant Mol Biol 12: 579-589
Ingelbrecht et al. (1994) Proc. Natl. Acad. Sci. USA 91, 10502-10506
Jefferson et al. (1987) EMBO J. 6, 3901-3907
Kawchuck et al. (1991) Molecular plant-microbe interactions 4, 247-253.
Keil et al. (1989) EMBO J. 8: 1323-1330
Keller et al. (1988)EMBO J. 7: 3625-3633
Keller et al.(1989)Genes Devel. 3: 1639-1646
Landsman et al. (1988) Mol Gen Genet 214, 68-73
Lindbo & Dougherty (1992a) Mol. Plant Micr. Int 5, 144-153
Lindbo & Dougherty (1992b) Virology 189, 725-733
Lindbo et al. (1993) Plant Cell 5, 1749-1759
Longstaff et al. (1993) EMBO J. 12, 379-386
MacDonald et al. (1991) Nucl. Acids Res. 19, 5575-5581
Metzlaff et al. (1997) Cell 88, 845-854
Meyer et al. (1987) Nature 330: 677
Mueller et al. (1995) Plant J. 7, 1001-1003
Ohta et al. (1990) Plant Cell. Physiol. 31, 805-813
Pang et al. (1996) Plant J. 9, 899-909
Peleman et al. 1989 Gene 84: 359-369
Powell-Abel et al. (1986) Science 232, 738-743
Powell et al. (1990) Virology 175, 124-130
Que et al. (1998) The Plant Journal 13, 401-409
Sambrook et al. (1989) Molecular Cloning: A Laboratory Manual, Second Edition,
Cold Spring Harbor Laboratory Press, NY
Sanford and Johnston, (1985) J. Theor. Biol. 113, 395-405
Schiebel et al. (1993a) Journal of Biological Chemistry 268: 11851-11857
Schiebel et al. (1993b) Journal of Biological Chemistry 268: 11858-11867
Smith et al. (1994) Plant Cell 6, 1441-1453
Stalberg et al., Plant Molecular Biol. 23, 671-683
Stam et al. (1997) Ann. Botan. 79, 3-12
Wagner and Sun (1998) Nature 391, 744-745
Wang et al. (1997) Journal of Genetics and Breeding 3
Wilbur and Lipmann (1983) Proc. Nat. Acad. Sci. U.S.A. 80: 726
Zheng et al. (1991) Plant Physiol. 97, 832-835
Zuker and Stiegler (1981) Nucl. Acids Res. 9, 133-148

## Claims

1. A method for reducing the phenotypic expression of a nucleic acid of interest, which can be expressed in a eukaryotic cell, comprising the step of introducing into said cell a chimeric RNA molecule comprising at least one RNA region with a nucleotide sequence comprising
i. a sense nucleotide sequence of at least 15 consecutive nucleotides having 100% sequence identity with at least part of the nucleotide sequence of the nucleic acid of interest; and
ii. an antisense nucleotide sequence including at least 15 consecutive nucleotides, having about 100% sequence identity with the complement of said at least 15 consecutive nucleotides of said sense nucleotide sequence;
wherein said RNA region is capable of forming an artificial hairpin RNA structure with a double stranded RNA stem by base-pairing between the regions with sense and antisense nucleotide sequence such that said at least 15 consecutive nucleotides of the sense sequence basepair with said at least 15 consecutive nucleotides of the antisense sequence,
wherein said nucleic acid of interest is a gene integrated into the genome of said eukaryotic cell,
wherein said RNA molecule is produced by transcription of a chimeric gene,
and wherein said method is not a method of treatment of the human or animal body by surgery or therapy, or a diagnostic method practised on the human or animal body.

2. A method for identifying a phenotype associated with the reduction of expression of a nucleic acid of interest in a eukaryotic cell, said method comprising
a. selecting within said nucleotide sequence of interest, a target sequence of at least 15 consecutive nucleotides;
b. designing a sense nucleotide sequence corresponding to the length of the selected target sequence and which has a sequence identity of 100% with said selected target sequence;
c. designing an antisense nucleotide sequence which comprises a stretch of at least 15 consecutive nucleotides with about 100% sequence identity to the complement of a part of said sense nucleotide sequence;
d. introducing a chimeric RNA molecule comprising both said sense and antisense nucleotide sequences into a plant cell comprising the nucleic acid of interest; and
e. observing the phenotype by a suitable method.

3. The method of claim 1 or 2, wherein the eukaryotic cell is a plant cell.

4. The method of claim 3, wherein said plant cell is comprised within a plant.

5. The method of claim 1 or 2 wherein said RNA molecule further comprises a spacer nucleotide sequence located between said sense and said antisense nucleotide sequences.

6. The method of claim 1 or 2 wherein the sense nucleotide sequence includes a sequence of 20 nucleotides with 100% sequence identity to the corresponding part of the nucleic acid of interest, and wherein the antisense nucleotide sequence includes a sequence of 20 nucleotides with 100% sequence identity to the complement of the sense nucleotide sequence.

7. The method of claim 1 or 2 wherein the sense nucleotide sequence includes a sequence of 50 nucleotides with 100% sequence identity to the corresponding part of the nucleic acid of interest, and wherein the antisense nucleotide sequence includes a sequence of 50 nucleotides with 100% sequence identity to the complement of the sense nucleotide sequence.

8. The method of claim 1 or claim 2, wherein said introducing of the chimeric RNA molecule into said plant cell is liposome-mediated.

9. A eukaryotic cell, comprising a nucleic acid of interest, which is normally capable of being phenotypically expressed, further comprising a chimeric RNA molecule comprising at least one RNA region with a nucleotide sequence comprising
i. a sense nucleotide sequence of at least 15 consecutive nucleotides having 100% sequence identity with at least part of the nucleotide sequence of the nucleic acid of interest; and
ii. an antisense nucleotide sequence including at least 15 consecutive nucleotides, having about 100% sequence identity with the complement of said at least 15 consecutive nucleotides of said sense nucleotide sequence;
wherein said RNA is capable of forming an artificial hairpin RNA with a double stranded RNA region by base-pairing between the regions with sense and antisense nucleotide sequence such that said at least 15 consecutive nucleotides of the sense sequence basepair with said at least 15 consecutive nucleotides of the antisense sequence,
wherein said nucleic acid of interest is a gene integrated into the genome of said eukaryotic cell.

10. The eukaryotic cell of claim 9 which is a plant cell.

11. The cell of claim 9 or 10 wherein said RNA molecule further comprises a spacer nucleotide sequence located between said sense and said antisense nucleotide sequences.

12. The cell of claim 9 or 10 wherein the sense nucleotide sequence includes a sequence of 20 nucleotides with 100% sequence identity to the corresponding part of the nucleic acid of interest, and wherein the antisense nucleotide sequence includes a sequence of 20 nucleotides with 100% sequence identity to the complement of the sense nucleotide sequence.

13. The cell of claim 9 or 10 wherein the sense nucleotide sequence includes a sequence of 50 nucleotides with 100% sequence identity to the corresponding part of the nucleic acid of interest, and wherein the antisense nucleotide sequence includes a sequence of 50 nucleotides with 100% sequence identity to the complement of the sense nucleotide sequence.

14. A plant comprising the plant cell of claim 9.


SEQUENCE LISTING

<110>  Commonwealth Scientific and Industrial Research
       Organisation
       Waterhouse, Peter M
       Wang, Ming-Bo
       Graham, Michael W

<120>  Methods and means for obtaining modified phenotypes

<130>  B07305BA-CA

<150>  US SN 09/056,767
<151>  1998-04-08

<150>  US SN 09/127,735
<151>  1998-08-03

<160>  7

<170>  PatentIn version 3.3

<210>  1
<211>  854
<212>  DNA
<213>  Potato virus Y

<400>  1
aagctttgaa gattgatttg atgccacata acccactcaa aatttgtgac aaaacaaatg      60

gcattgccaa atttcctgag agagagttcg agctaaggca gactgggcca gctgtagaag     120

tcgacgtgaa ggacatacca gcacaggagg tggaacatga agctaaatcg ctcatgagag     180

gcttgagaga cttcaaccca attgcccaaa cagtttgtag gctgaaagta tctgttgaat     240

atgggacatc agagatgtac ggttttggat ttggagcgta cataatagcg aaccaccatt     300

tgttcaggag ttataatggt tccatggagg tacgatccat gcacggtaca ttcagggtaa     360

agaatctaca cagtttgagc gttctgccaa ttaaaggtag ggacatcatc ctcattaaaa     420

tgccaaaaga tttccctgtc tttccacaga aattgcattt ccgagctcct acacagaacg     480

aaagaatttg tttagttgga accaactttc aggagaagta tgcatcgtcg atcatcacag     540

aagcaagcac tacttacaat ataccaggca gcacattctg gaagcattgg attgaaacag     600

ataatggaca ctgtggacta ccagtggtga gcactgccga tggatgtcta gtcggaattc     660

acagtttggc aaacaatgca cacaccacga actactactc agccttcgat gaagattttg     720

aaagcaagta cctccgaacc aatgagcaca atgaatgggt caagtcttgg atttataatc     780

cagacacagt gttgtggggc ccgttgaaac ttaaagacag cactcctaaa gggttattta     840

aaacaacaaa gctt                                                       854

<210>  2
<211>  2186
<212>  DNA
<213>  Artificial

<220>
<223>  coding region of the Gusd CoP construct


<220>
<221>  misc_feature
<223>  deficient Guus coding region

<220>
<221>  misc_feature
<223>  antisense to the 5′ end of the Gus coding region

<400>  2
atggtacgtc ctgtagaaac cccaacccgt gaaatcaaaa aactcgacgg cctgtgggca      60

ttcagtctgg atcgcgaaaa ctgtggaatt gatcagcgtt ggtgggaaag cgcgttacaa     120

gaaagccggg caattgctgt gccaggcagt tttaacgatc agttcgccga tgcagatatt     180

cgtaattatg cgggcaacgt ctggtatcag cgcgaagtct ttataccgaa aggttgggca     240

ggccagcgta tcgtgctgcg tttcgatgcg gtcactcatt acggcaaagt gtgggtcaat     300

aatcaggaag tgatggagca tcagggcggc tatacgccat ttgaagccga tgtcacgccg     360

tatgttattg ccgggaaaag tgtacgtatc accgtttgtg tgaacaacga actgaactgg     420

cagactatcc cgccgggaat ggtgattacc gacgaaaacg gcaagaaaaa gcagtcttac     480

ttccatgatt tctttaacta tgccggaatc catcgcagcg taatgctcta caccacgccg     540

aacacctggg tggacgatat ctacccgctt cgcgtcggca tccggtcagt ggcagtgaag     600

ggcgaacagt tcctgattaa ccacaaaccg ttctacttta ctggctttgg tcgtcatgaa     660

gatgcggact tgcgtggcaa aggattcgat aacgtgctga tggtgcacga ccacgcatta     720

atggactgga ttggggccaa ctcctaccgt acctcgcatt acccttacgc tgaagagatg     780

ctcgactggg cagatgaaca tggcatcgtg gtgattgatg aaactgctgc tgtcggcttt     840

aacctctctt taggcattgg tttcgaagcg ggcaacaagc cgaaagaact gtacagcgaa     900

gaggcagtca acggggaaac tcagcaagcg cacttacagg cgattaaaga gctgatagcg     960

cgtgacaaaa accacccaag cgtggtgatg tggagtattg ccaacgaacc ggatacccgt    1020

ccgcaaggtg cacgggaata tttcgcgcca ctggcggaag caacgcgtaa actcgacccg    1080

acgcgtccga tcacctgcgt caatgtaatg ttctgcgacg ctcacaccga taccatcagc    1140

gatctctttg atgtgctgtg cctgaaccgt tattacggat ggtatgtcca aagcggcgat    1200

ttggaaacgg cagagaaggt actggaaaaa gaacttctgg cctggcagga gaaactgcat    1260

cagccgatta tcatcaccga atacggcgtg gatacgttag ccgggctgca ctcaatgtac    1320

accgacatgt ggagtgaaga gtatcagtgt gcatggctgg atatgtatca ccgcgtcttt    1380

gatcgcgtca gcgccgtcgt cggtgaacag gtatggaatt tcgccgattt tgcgacctcg    1440

caaggcatat tgcgcgttgg cggtaacaag aaagggatct tcactcgcga ccgcaaaccg    1500

aagtcggcgg cttttctgct gcaaaaacgc tggactggca tgaacttcgg tgaaaaaccg 1560

cagcagggag gcaaacaatg aaacagacgc gtggttacag tcttgcgcga catgcgtcac 1620

cacggtgata tcgtccaccc aggtgttcgg cgtggtgtag agcatacgct gcgatggatt 1680

ccggcatagt taaagaaatc atggaagtaa gactgctttt tcttgccgtt ttcgtcggta 1740

atcaccattc ccggcgggat agtctgccag ttcagttcgt tgttcacaca aacggtgata 1800

cgtacacttt tcccggcaat aacatacggc gtgacatcgg cttcaaatgg cgtatagccg 1860

ccctgatgct ccatcacttc ctgattattg acccacactt tgccgtaatg agtgaccgca 1920

tcgaaacgca gcacgatacg ctggcctgcc caacctttcg gtataaagac ttcgcgctga 1980

taccagacgt tgcccgcata attacgaata tctgcatcgg cgaactgatc gttaaaactg 2040

cctggcacag caattgcccg gctttcttgt aacgcgcttt cccaccaacg ctgatcaatt 2100

ccacagtttt cgcgatccag actgaatgcc cacaggccgt cgagtttttt gatttcacgg 2160

gttgggtttt ctacaggacg taccat 2186

<210> 3
<211> 208
<212> DNA
<213> Artificial

<220>
<223> 5′ UTR of Johnson mosaic virus

<400> 3
cgccccgggc ccaacacaac acaacagaac ctacgtcaat tgattttatc aatcgcaaag 60

ccttacaaag atcttcgcag tcgttcatca acagattcac cgaaccattc ttgttagctc 120

tcgcacagag ataagcagga aaccatggca ggtgagtgga acacagtttg atagtaagag 180

aaaccagagg aagactgcag gtacccgc 208

<210> 4
<211> 1150
<212> DNA
<213> Artificial

<220>
<223> Subterranean clover virus S4 promoter with S7 enhancer

<400> 4
aatctgcagc ggccgcttaa tagtaattat gattaattat gagataagag ttgttattat 60

gcttatgagg aataaagaat gattaatatt gtttaatttt attccgcgaa gcggtgtgtt 120

atgtttttgt tggagacatc acgtgactct cacgtgatgt ctccgcgaca ggctggcacg 180

gggcttagta ttaccccgtg ccggatcaga gacatttgac taaatattga cttggaataa 240

tagcccttgg attagatgac acgtggacgc tcaggatctg tgatgctagt gaagcgctta 300

agctgaacga atctgacgga agagcggaca tacgcacatg gattatggcc cacatgtcta 360

aagtgtatct ctttacagct atattgatgt gacgtaagat gctttacttc gcttcgaagt 420

aaagtaggaa attgctcgct aagttattct tttctgaaag aaattattta attctaatta 480

aattaaatga gtcgctataa atagtgtcga tgctgcctca catcgtattc ttcttcgcat 540

cgtctgttct ggttttaagc gggatccagg cctcgagata tcggtacctt gttattatca 600

ataaaagaat ttttattgtt attgtgttat ttggtaattt atgcttataa gtaattctat 660

gattaattgt gaattattaa gactaatgag gataataatt gaatttgatt aaattaactc 720

tgcgaagcta tatgtctttc acgtgagagt cacgtgatgt ctccgcgaca ggctggcacg 780

gggcttagta ttaccccgtg ccgggatcag agacatttga ctaaatgttg acttggaata 840

atagcccttg gattagatga cacgtggacg ctcaggatct gtgatgctag tgaagcgctt 900

aagctgaacg aatctgacgg aagagcggac aaacgcacat ggactatggc ccactgcttt 960

attaaagaag tgaatgacag ctgtctttgc ttcaagacga agtaaagaat agtggaaaac 1020

gcgtaaagaa taagcgtact cagtacgctt cgtggcttta tataaatagt gcttcgtctt 1080

attcttcgtt gtatcatcaa cgaagaagtt aagctttgtt ctgcgtttta atgatcgatg 1140

gccagtcgac 1150

<210> 5
<211> 1052
<212> DNA
<213> Artificial

<220>
<223> Subterranean clover virus S4 promoter with S4 enhancer

<400> 5
ggatccaggc ctcgagatat cggtaccttg ttattatcaa taaaagaatt tttattgtta 60

ttgtgttatt tggtaattta tgcttataag taattctatg attaattgtg aattattaag 120

actaatgagg ataataattg aatttgatta aattaactct gcgaagctat atgtctttca 180

cgtgagagtc acgtgatgtc tccgcgacag gctggcacgg ggcttagtat taccccgtgc 240

cgggatcaga gacatttgac taaatgttga cttggaataa tagcccttgg attagatgac 300

acgtggacgc tcaggatctg tgatgctagt gaagcgctta agctgaacga atctgacgga 360

agagcggaca aacgcacatg gactatggcc cactgcttta ttaaagaagt gaatgacagc 420

tgtctttgct tcaagacgaa gtaaagaata gtggaaaacg cgtggatcca ggcctcgaga 480

tatcggtacc ttgttattat caataaaaga atttttattg ttattgtgtt atttggtaat 540

ttatgcttat aagtaattct atgattaatt gtgaattatt aagactaatg aggataataa 600

ttgaatttga ttaaattaac tctgcgaagc tatatgtctt tcacgtgaga gtcacgtgat 660

gtctccgcga caggctggca cggggcttag tattaccccg tgccgggatc agagacattt 720

gactaaatgt tgacttggaa taatagccct tggattagat gacacgtgga cgctcaggat 780

ctgtgatgct agtgaagcgc ttaagctgaa cgaatctgac ggaagagcgg acaaacgcac 840

atggactatg gcccactgct ttattaaaga agtgaatgac agctgtcttt gcttcaagac 900

gaagtaaaga atagtggaaa acgcgtaaag aataagcgta ctcagtacgc ttcgtggctt 960

tatataaata gtgcttcgtc ttattcttcg ttgtatcatc aacgaagaag ttaagctttg 1020

ttctgcgttt taatgatcga tggccagtcg ac 1052

<210> 6
<211> 1583
<212> DNA
<213> Artificial

<220>
<223> coding sequence of the desaturase CoP construct

<220>
<221> misc_feature
<222> (1)..(480)
<223> corresponding to the 5' end of the delta12-desaturase (fad2)
 coding region in sense orientation

<220>
<221> misc_feature
<222> (1101)..(1583)
<223> corresponding to the 5' end of the delta12-desaturase (fad2)
 coding region in anti sense orientation

<400> 6
atcattatag cctcatgctt ctactacgtc gccaccaatt acttctctct cctccctcag 60

cctctctctt acttggcttg gccactctat tgggcctgtc aaggctgtgt cctaactggt 120

atctgggtca tagcccacga atgcggtcac cacgcattca gcgactacca atggctggat 180

gacacagttg gtcttatctt ccattccttc ctcctcgtcc cttacttctc ctggaagtat 240

agtcatcgcc gtcaccattc caacactgga tccctcgaaa gagatgaagt atttgtccca 300

aagcagaaat cagcaatcaa gtggtacggg aaatacctca acaaccctct tggacgcatc 360

atgatgttaa ccgtccagtt tgtcctcggg tggcccttgt acttagcctt taacgtctct 420

ggcagaccgt atgacgggtt cgcttgccat ttcttcccca acgctcccat ctacaatgac 480

cgagaacgcc tccagatata cctctctgat gcgggtattc tagccgtctg ttttggtctt 540

taccgttacg ctgctgcaca agggatggcc tcgatgatct gcctctacgg agtaccgctt 600

ctgatagtga atgcgttcct cgtcttgatc acttacttgc agcacactca tccctcgttg 660

cctcactacg attcatcaga gtgggactgg ctcaggggag ctttggctac cgtagacaga 720

gactacggaa tcttgaacaa ggtgttccac aacattacag acacacacgt ggctcatcac 780

ctgttctcga caatgccgca ttataacgca atggaagcta caaaggcgat aaagccaatt 840

ctgggagact attaccagtt cgatggaaca ccgtggtatg tagcgatgta tagggaggca 900

aaggagtgta tctatgtaga accggacagg gaaggtgaca agaaaggtgt gtactggtac 960

aacaataagt tatgagcatg atggtgaaga aattgtcgac ctttctcttg tctgtttgtc 1020

ttttgttaaa gaagctatgc ttcgttttaa taatcttatt gtccattttg ttgtgttatg 1080

acattttggc tgctcattat gttcagtaac atctaccctc gcaacccctt ctttaccgtt 1140

cgcttgggca gtatgccaga cggtctctgc aatttccgat tcatgttccc ggtgggctcc 1200

tgtttgacct gccaattgta gtactacgca ggttctccca acaactccat aaagggcatg 1260

gtgaactaac gactaaagac gaaaccctgt ttatgaagta gagaaagctc cctaggtcac 1320

aaccttacca ctgccgctac tgatatgaag gtcctcttca ttccctgctc ctccttcctt 1380

accttctatt ctggttgaca cagtaggtcg gtaaccatca gcgacttacg caccactggc 1440

gtaagcaccc gatactgggt ctatggtcaa tcctgtgtcg gaactgtccg ggttatctca 1500

ccggttcggt tcattctctc tccgactccc tcctctctct tcattaacca ccgctgcatc 1560

atcttcgtac tccgatatta cta 1583

<210> 7
<211> 786
<212> DNA
<213> Artificial

<220>
<223> intron 2 of the Flaveria trinervia puryvate orthophosphate
      dikinase

<400> 7
aagcttggta aggaaataat tattttcttt tttcctttta gtataaaata gttaagtgat 60

gttaattagt atgattataa taatatagtt gttataattg tgaaaaaata atttataaat 120

atattgttta cataaacaac atagtaatgt aaaaaaatat gacaagtgat gtgtaagacg 180

aagaagataa aagttgagag taagtatatt atttttaatg aatttgatcg aacatgtaag 240

atgatatact agcattaata tttgttttaa tcataatagt aattctagct ggtttgatga 300

attaaatatc aatgataaaa tactatagta aaaataagaa taaataaatt aaaataatat 360

ttttttatga ttaatagttt attatataat taaatatcta taccattact aaatatttta 420

gtttaaaagt taataaatat tttgttagaa attccaatct gcttgtaatt tatcaataaa 480

caaaatatta aataacaagc taaagtaaca aataatatca aactaataga aacagtaatc 540

taatgtaaca aaacataatc taatgctaat ataacaaagc gcaagatcta tcattttata 600

tagtattatt ttcaatcaac attcttatta atttctaaat aatacttgta gttttattaa 660

cttctaaatg gattgactat taattaaatg aattagtcga acatgaataa acaaggtaac 720

atgatagatc atgtcattgt gttatcattg atcttacatt tggattgatt acagttggga 780

aagctt 786